(19) Europäisches Patentamt
European Patent Office
Office européen des brevets

(11) Publication number: **0 206 200 B1**

(12) **EUROPEAN PATENT SPECIFICATION**

(45) Date of publication of patent specification: **23.09.92**

(51) Int. Cl.5: **C12N 15/55**, C12P 21/00, C12N 9/18, G01N 33/573, C12Q 1/68, A61K 37/54, C12N 1/00, C12N 1/20

(21) Application number: **86108189.1**

(22) Date of filing: **16.06.86**

(54) **Human cholinesterase-type proteins and their production.**

(30) Priority: **18.06.85 IL 75553**

(43) Date of publication of application:
**30.12.86 Bulletin 86/52**

(45) Publication of the grant of the patent:
**23.09.92 Bulletin 92/39**

(84) Designated Contracting States:
**AT BE CH DE FR GB IT LI LU NL SE**

(56) References cited:
**EP-A- 0 001 930**
**EP-A- 0 114 756**
**US-A- 2 475 793**

**Proc. Natl. Acad. Sci. USA, vol. 8ö2, March 1985, pages 1827-1831, H. Soreq et al "A human acetylcholinesterase gene identified by homology to the Ace region of Drosophila"**

(73) Proprietor: **YEDA RESEARCH AND DEVELOPMENT COMPANY LIMITED**
**P.O. Box 95**
**Rehovot 76100(IL)**

(72) Inventor: **Soreq, Hermona**
**8 Hahermon Street**
**Rishon-Le-Zion(IL)**

(74) Representative: **Vossius & Partner**
**Siebertstrasse 4 P.O. Box 86 07 67**
**W-8000 München 86(DE)**

**EP 0 206 200 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Chemical abstracts, vol. 103 n 3, July 22, 1985, page 148, ref. n 17703z, Columbus, Ohio, US, H. Soreq et al "Human acetylcholinesterase gene is homologous to the Ace locus in Drosophila" & Symp. Giovanni Lorenzini Found. 1984, 20 (Dev. Neurosci.), 95-9

Chemical abstracts, vol. 101 n 25, December 1984, page 362, ref. n 225808y, Columbus, Ohio, US & CS-A-214543 (J. Zabransky et al), 20.1.1981 and ref. n 225809z & CS-A-214546 (J. Zemek et al) 28.2.1984

Proc. Natl. Acad. Sci. USA, vol. 79, February 1982, US, pages 830-834 H. Soreq et al "Biosynthesis and secretion of catalytically active acetylcholinesterase in Xenopus ocytes microinjected with mRNA from rat brain and from Torpedo electric organ" and pages 1078-1082, D.M. Fambrough et al "Acetylcholinesterase of human erythrocytes and neuromuscular junctions : homologies revealed by monoclonal antibodies"

Proc. Natl. Acad. Sci. USA, vol. 80, January 1983, pages 278-282, US, R.J. Conner et al "Detection of sickle cell betaS-globin allele by hybridization with synthetic oligonucleotides"

## Description

### FIELD OF THE INVENTION:

The invention is in the field of the neurotransmitter-hydrolyzing enzyme, acetylcholinesterase, EC 3,1.1.7, designated as AChE, which is a key element in cholinergic synapses and in neuromuscular junctions. Cholinesterases (ChEs) appear in different cell types, where they exist in multiple molecular forms. According to the invention, several types of cDNA and mRNA are provided which code for the main types of human ChE. Genetic engineering was used to provide sources for the large scale production of various ChE types. There are provided expression systems, capable of producing such ChE.

A ChE thus made available, is of value in the treatment of organophosphorus (OP) poisoning, as antidote and for the treatment of patients poisoned by such poisonous substances.

Synthetic oligonucleotide probes prepared according to the consensus hexapeptide sequence present in the organophosphate binding site of cholinesterases is provided. Such probes are used to select cDNA fragments containing such sequence from genetically engineered libraries of various sources.

There are further provided bacterial colonies containing such cDNA fragments, inserted into suitable expression vectors. When suitably induced to produce the peptide encoded by the cloned DNA, these cells synthesize proteins immunoreactive with anti-AChE antibodies. Thus identification of mammalian DNA sequences directly related to ChE biosynthesis was effected.

Furthermore, the invention relates to protein having authentic ChE-type catalytic activity. Such proteins are effective ingredients in succinylcholine and/or organophosphorus anticote preparations.

There are provided antibodies interacting with human AChE.

### BACKGROUND OF THE INVENTION:

#### Structural and Physiological Properties of Cholinesterases

At the cholinergic synapse, the enzyme acetylcholinesterase (acetylcholine hydrolase, AChE, EC 3.1.1.7) terminates the electrophysiological response to the neurotransmitter acetylcholine (ACh) by degrading it very rapidly. (For a review see Silver, A., The Biology of Cholinesterases. North-Holland Pub. Co., Amsterdam (1974)).

Mammmalian cholinesterases (ChEs) exhibit extensive polymorphism at several levels. They can be distinguished by substrate specificity into acetylcholinesterase (acetylcholine hydrolase, EC 3.1.1.7, AChE) and pseudocholinesterase (acylcholine acylhydrolase, EC 3.1.1.8, $\psi$ChE).

They differ in their susceptibility to various inhibitors. Both are composed of subunits of ca.600 amino acid residues each, and are glycosylated (10-20%).

ChEs occur in multiple molecular forms, which exhibit different sedimentation coefficients on sucrose gradients, display different hydrodynamic interactions with non-ionic detergents and are composed of different numbers of subunits. (For a comprehensive review see Massoulie, J. and Bon, S., Ann. Rev. Neurosci. 5, 57-106, (1982)).

There are secreted, cytoplasmic and membrane-associated pools of ChEs in the mammalian nervous tissue, but all forms of ChEs possess similar catalytic properties, suggesting that they share common acetylcholine binding sites (Chubb, I.W., In: Cholinesterases - Fundamental and Applied Aspects. M. Brzin, E.A. Barnard and D. Sket, Eds. pp. 345-359, (1984)). But various ChEs may also contain distinct polypeptide regions, responsible for the subcellular segregation of various AChE forms, for their different amphipathic properties and for their different modes of assembly into multisubunit protein molecules. This is supported by the reports of antibodies which display homologies (Fambrough et al., Proc. Natl. Acad. Sci. USA 79, 1078-1082, (1982)); as well as differences (Doctor et al., Proc. Natl. Acad. Sci. USA 80, 5767-5771, (1983)) between different forms of AChE from various organisms.

#### Interaction of Cholinesterases with Organophosphorous Insecticides and Nerve Gases.

Most of the commonly used insecticides are organophosphorus (OP) compounds, acting by blocking the insect's AChE. In many cases where such insects develop insensitivity to the insecticides, this occurs because of the enhanced expression of AChE forms which do not interact with the organophosphate. To deal with such phenomenae successfully, it is necessary to know the mechanisms by which the appearance of such AChE forms is regulated.

Complete inhibition of mamalian AChE (i.e., by administration of OP-poisons) is lethal, due to the formation of a stable stoichiometric (1:1) covalent conjugate with the active site serine. Treatment of OP poisoning has involved the use of reversible ChE inhibitors, and reactivation of the inhibited enzyme with active-site directed nucleophiles (e.g., quaternary oximes) which detach the phosphoryl moiety from the hydroxyl group of the active site serine. A parallel competing reaction, termed "aging", transforms the inhibited ChE into a form that cannot be regenerated by the commonly used reactivators by dealkylation of the covalently bound OP group, and renders therapy of intoxication by certain organophosphates, such as Sarin, DFP, and Soman, exceedingly difficult (National Academy of Sciences Report, 1982).

## Frequent Mutations in Cholinesterase Genes

Pseudocholinesterase (ψChE) is particularly enriched in the serum. Subjects with "null" ψChE-Activity (i.e., samples of their serum cannot hydrolyze acetylcholine) do not exhibit any health problem under natural conditions, but suffer from prolonged apnea when they are given succinylcholine (a drug commonly used as a short-acting muscle relaxant) in surgical operation. However, there are no rapid, simple, routine methods to detect and characterize the atypic forms of the enzyme prior to surgery.

The amino acid sequence of human AChE is not known. It has recently been found that the same 6 amino acids are included in the organophosphorous binding site of both Torpedo AChE tetramers and human pseudoChE tetramers. (McPhee-Quigley, K, J. Biol. Chem. 260 12185-12189 (1985); Schumacher et al., Nature 319, 407-409 (1986)).

The amino acid sequence in this hexapeptide from the organophosphate-binding site of ChEs has been determined recently in several laboratories, by partial proteolytic digestion of the purified enzymes, labeled with [$^3$H]-diisopropylfluorophosphate, and was found to be Gly-Glu-Ser-Ala-Gly-Ala, with the serine residue bound to organophosphates. It has been suggested that the "null" forms ψChE are modified in this peptide.

## Common Alterations in the Level and Properties of Cholinesterases

Modifications in both the level (Spokes, E.G.S., Brain 103, 179-183, (1980)) and the composition of molecular forms (Atack J.R. et al., Neurosci. Lett. 40, 199-204, (1983)) of human brain AChE have been reported in several neurological or genetic disorders, such as Senile Dementia of the Alzheimer's type (SDAT; about 5% of the population above 65), or Downs syndrome (trisomy of chromosome 21). The level of AChE in cholinergic brain areas drops by about 50%, and the tetrameric form of the enzyme disappears completely in SDAT.

Individuals with Down's syndrome invariably develop SDAT manifestations before the age of 40. In addition open neural tube defects in human embryos are clinically characterized by secretion of AChE tetramers into the amniotic fluid. Current tests for these phenomenae include sucrose gradient fractionation, followed by enzymatic assays of substrate hydrolysis or gel electrophoresis and AChE activity staining.

Missing, and particularly desirable, are simple selective procedures to determine the level of specific AChE forms.

Homozygote Drosophila mutants lacking the Ace locus which controls AChE biosynthesis, die at a very early stage of larval development (Hall, J.O., Quar. Rev. Biophysics 15, 3-479, (1982)). Nematode mutants defective in the expression of one of their two cholinesterase genes cannot survive. It is highly likely that homozygous mutations in AChE genes in humans will result in early abortion or in severe neuromuscular malformations in the fetus. So far, there are no methods to determine whether specific individuals carry such mutations.

## SUMMARY OF THE INVENTION

The present invention relates to genetically engineered AChE-like enzymatically active peptides. More particularly the invention relates to such peptides which react with polyclonal and monoclonal acetylcholinesterase-specific antibodies.

The invention furthermore relates to cDNA coding for a genetically engineered protein defined above, to expression systems as well as to mRNA coding for mammalian ChE in a synthetic form. The cDNAs contain nucleotide sequences as herein defined; the invention further relates to fragments of a human cholinesterase gene containing the nucleotide sequences coding for human cholinesterases and to such fragments cloned in an expression vector such as a suitable phage, (Charon 4A phage) or the like.

## DEPOSIT IN INTERNATIONAL CULTURE COLLECTION:

Phage FBChE 12a λgt10 and plasmid FBChEpEX 12a were deposited with the Culture Collection of Institute Pasteur, Paris, France under Deposit No. I-534 and I-552, respectively, with instructions complying with those required for a patent application.

Other and further features of the invention will become apparent from the following description.

Amongst applications of the products of the invention there may be mentioned:

1. Utility: the present invention provides means for producing ample quantities of the enzyme which binds to organophophorus poisons with a very high Kd value. This protein (or part of it) can be used as a highly effective agent against OP poisoning.

2. Clinical detection of ψChE deficiencies will prevent the post-surgery prolonged apnea phenomenon, using oligonucleotide hybridization to genomic DNA, similar to the detection of abnormalities in the gene coding for sickle cell beta-S globin (Conner, B.J. et al., Proc. Natl. Acad. Sci. USA 80, 278-282, (1983)). There is provided an assay adapted to distinguish between these normal and defective sequences in minute samples of genomic DNA and in a single hybridization step.

3. Elucidation of the active site topography and the amino acid sequence of AChE opens up new approaches for the development of rapid, simple clinical methods to detect poisoning or disease-related changes in ChEs. This can be done, for example, by radioimmunoassay, using monospecific antibodies elicited against peptide domains that are specific to the tetrameric form, which appears to be the major one to be altered in various neurological disorders.

4. If indeed it will be proved that mutations in ChE gene(s) are lethal or cause severe damage, a method to detect such mutations at the level of DNA can be provided, which enables the defective genes to be identified at a very early gestational stage. This can be done by hybridization tests, using DNA from chorionic villi or amniotic fibroblasts and well-characterized probes from ChE gene(s).

## Description of the Preferred Embodiment

To identify the mRNA species coding for human ChEs, we developed the use of microinjected *Xenopus* oocytes as a highly sensitive and efficient translation system, which is capable of producing catalytically active ChEs (Soreq, H. et al., Proc. Natl. Acad. Sci. USA 79, 830-835, (1982)).

Injection of mRNA from different ChE-expressing sources into these oocytes produces ChE activities (Soreq, H. et al., EMBO Journal 3, 1371-1375, (1984)). This bioassay issued to directly monitor the levels of ChE mRNA in the corresponding tissue. In combination with our recently developed methods for measuring ChE activity (Parvari, R. et al., Anal. Biochem. 133, 450-456 (1983)), we can now determine these activities with a particularly high sensitivity and also define the biochemical properties of the oocyte-produced enzyme (see Soreq, H. ORC Critical Reviews in Biochem. Vol. 18, 199-238, (1985) for a recent review).

Determination of ChE mRNA levels in the oocytes was proven to be time and concentration dependent and the enzyme produced from brain poly(A)$^+$ RNA was biochemically defined as "true" mammalian AChE. When the concentration of ChE mRNA out of total poly(A)$^+$ RNA was calculated, it yielded values of about $1x10^{-5}$ of total mRNA. (Soreq et al., ibid. (1984)).

Sucrose gradient fractionation of DMSO-denatured mRNA from primary gliomas, meningiomas and embryonic brain revealed three size classses of ChE-inducing mRNAs, sedimenting at 32S, 20S and 9S. The amounts of these different classes of ChE-inducing mRNAs varied between the three tissue sources examined. To distinguish between ChEs produced in occytes and having different substrate specificities, their activity was determined in the presence of selective inhibitors. Both AChE and ψChE multimeric cholinesterase activities were found in the mRNA-injected oocytes. Moreover, human brain mRNAs inducing 'true' and 'pseudo' ChE activities had different size distribution, indicating that different mRNAs might be translated into various types of ChEs.

These findings imply that the heterogeneity of ChEs in the human nervous system is not limited to the post-translational level, but extends to the level of mRNA. Furthermore, the composition of ChE mRNAs appears to be different in various brain cell types, and the production of the differently migrating ChE molecular forms thus seems to be controlled at least partially at the level of mRNA.

To ensure a complete representation of all of the ChE mRNA sequences in different cell types in the brain, it was necessary to prepare the cDNA library from the entire brain. Therefore, we could not take advantage of the fact that particular brain regions are highly enriched with AChE. Our mRNA preparation of choice was, therefore, total, unfractionated poly(A)$^+$ mRNA from human fetal brain. To focus on the particular DNA sequences which actually code for the ChE proteins, it was decided to search for probes of high specificity and selectivity.

Such properties could be offered by synthetic short oligonucleotides, prepared according to amino acid sequence(s) that are unique to cholinesterases.

Synthetic oligodeoxyribonucleotides have been shown to hybridize specifically to complementary DNA sequences (Wallace, R.B. et al., Nuc. Acids. Res. 9, 879-894, (1981)). Under appropriate hybridization conditions, only well base-paired oligonucleotide xDNA duplexes will form; duplexes containing mismatched base pairs will not be stable.

This technique has been applied to the successful isolation of cDNAs coding for human $beta_2$ macroglobulin, for murine transplantation antigen, for human apolipoprotein and many others.

The consensus hexapeptide sequence from the organophosphate-binding site of ChEs (designated OPSYN) is Phe-Gly-Glu-Ser-Ala-Gly. Because of the ambiguity in the genetic code, this peptide could be encoded by one out of 384 different oligonucleotides, as follows:

| OPSYN oligonucleotides | | | | | |
|---|---|---|---|---|---|
| Phe<br>AAG<br>A | Gly<br>CCN | Glu<br>CTC<br>T | Ser<br>AGN<br>TCA<br>C | Ala<br>CGN | Gly<br>CC |
| (where N = A, C, G or T). | | | | | |

Three mixtures of these oligodeoxynucleotides, each containing 128 different chains of 17 nucleotides each, designated OPSYN, were prepared by solid phase synthesis using phosphoramidite chemistry (Beaucage et al., Tetra. Lett. 22, 1859-1862 (1981); Mcbride et al., Tetra. Lett. 24, 245-248 (1983); Atkinson et al., Oligonucleotide synthesis - a practical approach, Gate M.J. (ed), IRL Press, 35-81 (1984)). The synthetic procedure employed was essentially that given by Adams et al., J. Am. Chem. Soc., 105, 661-663 (1983). In synthetic cycles where mixtures of two or four nucleotides had to be introduced, the solid support was divided into two or four equal parts. Each of the portions was reacted with only one of the required phosphoramidite reagents. Following this cycle, the various portions were recombined.

This procedure ensured the presence, in the mixture, of roughly equal amounts of each of its components. The oligodeoxynucleotides were purified by polyacrylamide gel electrophoresis (20% acrylamide, 7 M urea), followed by Sephadex G-50 chromatography.

The OPSYN probes happen to be particularly rich in guanosine and cytosine residues. We therefore carried out the hybridization with thses probes at 40°C, to prevent high non-specific binding. Phage DNA was transferred from plaques to nitrocellulose filters (Maniatis et al., Molecular cloning, a laboratory manual. Cold Soring Harbor Laboratory Press (1982)). Filters were incubated for prehybridization in 6 x SSC (1 x SSC = 0.015 M sodium citrate and 0.15 M NaCl), 5 x Denhardt (1 x Denhardt's solution is 0.02% Ficoll/0.02% polyvinylpyrollidone/0.02% bovine serum albumin) and 0.1 mg/ml salmon sperm DNA for 2 h at 40°C. This step was followed by hybridization in 6 x SSC, 5 x Denhardt, 0.05 mg/ml salmon sperm DNA and 3 x $10^6$ dpm/ml of [$^{32}$P]-labeled oligonucleotide probe, for 16 h at 40°C. Filters were then washed at 40°C in 3 x SSC, 40 min each wash with 4-8 changes of washing solution until released radioactivity decreased below 300 dpm/ml. Exposure duration was 1-3 days with intensifying screens (CAWO) (2-3 days for first screen, 1 day for further screens).

Following the third screening step, 3 M tetramethylammonium chloride $(CH_3)_4NCl$) was employed to discriminate against short, GC-rich hybrids in a base composition-independent manner (Wood et al., Proc. Natl. Acad Sci, U.S.A. 82, 1585-1588 (1985)). Filters were first rinsed at 37°C with 3 N $(CH_3)_4NCl$ solution, containing 50 mM Tris HCl, pH 8.0, 2 mM EDTA and sodium dodecyl sulfate at 1 mg/ml. Rinsed filters were washed twice in a shaking bath for 20 min with the same $(CH_3)_4NCl$ solution at 53°C ± 1°C.

To increase the certainty of our search, we prepared an additional oligodeoxynucleotide probe. This probe, designated OPSYNO, consisted of a mixture of thirty-six oligodeoxynucleotides, each 29-nucleotides-long. These encoded a decapeptide comprising the same hexapeptide as the OPSYN probes and 4 additional amino acids, Ala-Ala-Ser-Val, as reported for the continuation of this peptide in human serum ψChE (Lockeridge, O "Cholinesterases-Fundamental and Applied Aspects" edited Brzin et al., pp5-12 (1984)). To limit the complexity of this probe and ensure that the correct oligodeoxynucleotide would be present in sufficient specific activity, we inserted deoxyinosine in every position where all four nucleotides might be found. Deoxyinosine, being an "inert" nucleotide, is expected either to hybridize with A, C or T or at least not to interfere with the stability of hybridization (Takahashi et al., Proc. Natl. Acad. Sci. U.S.A. 82, 1931-1935 (1985)). However, the stability of such non-perfect hybrids, containing deoxyinosine, is expected to be lower than that of perfect hybrids of the same length (Takahashi ibid. (1985)). This implies that to remain stable under our washing conditions, OPSYNO x cDNA hybrids should contain more than 17 base pair matches. In other words, OPSYN-positives with 16-17 base pair matches that do not represent the correct cDNA can be excluded with the aid of OPSYNO.

Altogether, four screening experiments were performed; these resulted in the isolation of 8 $(CH_3)_4NCl$-stable OPSYN positives, out of which only a single clone hybridized with OPSYNO as well. Furthermore, the hybridization signals displayed by this clone with both OPSYN I and II and OPSYNO were the only ones that remained stable to 3 M $(CH_3)_4NCl$ washes. The isolated cDNA insert, designated FBChEI2, consisted of 765 nucleotides with an open reading frame sufficient to code for about half of the subunit size of human cholinesterase (Lockridge, ibid. (1984)).

The nucleotide sequence of FBChEI2 that is complementary to probes OPSYN and OPSYNO corresponded exactly to the peptide sequence used to design these oligodeoxynucleotide probes. When the amino acids predicted from FBChEI2 sequence are aligned with the available peptide sequences published for human ψChE, about half of the coding region for the mature enzyme is defined, starting at residue 1 (nucleotide 187), which corresponds to the N-terminal peptide, and ending at residue 202, which is included in the active site tryptic peptide of ψChE as determined from amino acid sequencing. The sequence includes the active site serine, which can be labeled by diisopropylfluorophosphate (Lockridge, ibid. (1984)). The N-terminal peptide inferred by the FBChEI2 sequence is similar to the ψChE peptide, except that in our sequence residue 12 is Lys, as in the N-terminal peptide of Torpedo AChE (McPhee-Quigley et al., ibid. (1985)), whereas the corresponding residue of human ψChE found by amino acid sequencing is Gly. In addition, the N-terminus of the ChE encoded by FBChEI2 differs from the peptide reported for erythrocyte AChE (Haas and Rosenberry, Anal, Biochem. 148, 154-162 (1985)). Altogether, this suggests that FBChEI2 codes for ψChE.

The region upstream of the ψChE amino-terminal residue (nucleotides 115-187) in FBChEI2 codes for 20 amino acids characteristic of leader peptides of membrane-associated and exported protein precursors. The hydrophobic sequence in this region is rich in large nonpolar amino acids. It is preceded by the tripeptide His-Ser-Lys, and terminates with Lys-Ser-His, both composed of polar amino acids. Further upstream, the cDNA sequence consists of a fully open reading frame without stop codons and includes two methionine residues.

The DNA and inferred amino acid sequence of the FBChEI2 clone were compared to the parallel sequences recently published for a cDNA clone coding for AChE from Torpedo electric organ (Schumacher et al., ibid. (1986)). This analysis revealed a 47% homology between the corresponding parts of the Torpedo and the human enzymes, strongly suggesting that they have a common ancestral origin. A higher level of conservation was found at the amino acid level than at the DNA level.

For direct characterization of the protein encoded by FBChEI2, the cDNA insert was subcloned into the pEX bacterial expression vector in conjunction with the gene coding for β-galactosidase. Induction of pEX expression resulted in the production of two polypeptides, of 90 and 40 kilodalton, which reacted with anti β-galactosdidase antibodies, while the synthesis of intact β-galactosidase (117 kilodalton) ceased almost completely. The 40 kd polypeptide, but not the 90 kd one, interacted specifically in protein blots with antibodies against both human erythrocyte and Torpedo electric organ AChE, suggesting that both polypeptides are derived from a partially proteolyzed fusion protein of β-galactosidase-FBChEI2, with the 40 kd polypeptide containing the information encoded by FBChEI2. Protein blot analysis with antiserum against whole human serum proteins failed to reveal a significant specific interaction (not shown), perhaps due to the low amount of anti-cholinesterase antibodies in this polyspecific antiserum.

The bacterial-produced protein encoded in pEX vector by this human ChE cDNA was purified by preparative gradient gel elecrophoresis, electroelution and lyophilization and was injected into rabbits together with complete Freund's adjuvant. Rabbit serum was prepared following three successive injections of 300 ug of this protein at two week intervals. Immunoblot analysis revealed that this serum contains antibodies that interact at 1:10,000 diltuion with amounts as low as 20 ng of highly purified 70 Kd AChE from erythrocytes. It also interacted with proteins of ca. 90 Kd in extracts from fetal brain and liver, both rich in AChE, and of ca. 60 Kd in fetal muscle and liver, both rich in ψChE. (Antibodies to Human Cholinesterases Elicited by Bacterial-Expressed Products of Human Cholinesterase cDNA, Soreq et al., Abst. Neurosci. Soc., in press (1986)).

This antiserum can be employed for a clinical assay for determining changes in the levels and properties of ChEs such as in Alzheimer's disease patients.

To study the in vivo biosynthesis of the protein encoded by FBChEI2, the insert cDNA was [$^{32}P$]-labeled and hybridized with human RNA and DNA. In RNA blots loaded with 10 μg of poly(A)$^+$RNA/lane, [$^{32}P$]-labeled FBChEI2 interacted with a single 2.4 kb band of RNA that was present in fetal brain and liver, but not in the cholinesterase-deficient Human Epidemoid carcinoma (Soreq et al., ibid. (1984)). The exposure time needed to visualise the hybridization was relatively long (10 days), suggesting that this mRNA exists at rather low concentrations even in positive tissue sources such as fetal brain and liver. This assumption was also corroborated by re-screening the fetal brain library with [$^{32}P$]-labeled FBChEI2. Six out of 1 x $10^6$ phages contained three different inserts that hybridized with FBChEI2.

Two of these were partial fragments included in FBChEI2 and the third displayed only partial sequence homology with the original cDNA clone.

This analysis emphasizes the scarcity of cholinesterase cDNA clones in the cDNA library.

The levels of the mRNAs coding for particular types of cholinesterase in fetal brain and liver were analyzed in parallel by mRNA microinjection into Xenopus ooyctes, where AChE mRNA and ψChE mRNA are translated to yield their catalytically active enzyme products.

Considerable production of iso-OMPA-insensitive AChE was observed in oocytes injected with either fetal brain of liver RNA but not with HEpRNA. In contrast, only liver mRNA was capable of producing significant levels of BW284C51-insensitive ψChE. Thus, the pattern revealed in the RNA blot hybridization is compatible with the levels of AChE mRNA, or with both species of cholinesterase mRNA together, but not with pseudo-ChE mRNA alone.

The genomic DNA origin of FBChEI2 was examined by DNA blot hybridization. [$^{32}P$]-labeled FBChEI2 hybridized with two human DNA fragments derived by restriction with EcoRI, 4.7 and 2.5 kb in length.

Taking into account the existence of an internal EcoRI site in the original FBChEI2 cDNA clone, and comparing the signal intensity observed with 20 μg of genomic DNA to that detected with 1.0 ng of λgt10-FBChEI2, this analysis indicates that the DNA sequences hybridizing with FBChEI2 are not present in the human genome in many copies. Parallel analysis of mouse DNA revealed a significant but not intense hybridization, suggesting the existence of homologous cholinesterase DNA sequences in other mammalian species.

Accordingly there is also provided a clone designated as FBChE12a which has the translated sequence:

```
ATT TCC CCG AAG TAT TAC ATG ATT TTC ACT CCT  33
Ile Ser Pro Lys Tyr Tyr Met Ile Phe Thr Pro
TGC AAA CTT TGC CAT CTT TGT TGC AGA GAA TCG  66
Cys Lys Leu Cys His Leu Cys Cys Arg Glu Ser
GAA ATC AAT ATG CAT AGC AAA GTC ACA ATC ATA  99
Glu Ile Asn Met His Ser Lys Val Thr Ile Ile
TGC ATC AGA TTT CTC TTT TGG TTT CTT TTG CTC 132
Cys Ile Arg Phe Leu Phe Trp Phe Leu Leu Leu
TGC ATG CTT ATT GGG AAG TCA CAT ACT GAA GAT 165
Cys Met Leu Ile Gly Lys Ser His Thr Glu Asp
GAC ATC ATA ATT GCA ACA AAG AAT GGA AAA GTC 198
Asp Ile Ile Ile Ala Thr Lys Asn Gly Lys Val
AGA GGG ATG AAC TTG ACA GTT TTT GGT GGC ACG 231
Arg Gly Met Asn Leu Thr Val Phe Gly Gly Thr
GTA ACA GCC TTT CTT GGA ATT CCC TAT GCA CAG 264
Val Thr Ala Phe Leu Gly Ile Pro Tyr Ala Gln
CCA CCT CTT GGT AGA CTT CGA TTC ACA AAG CCA 297
Pro Pro Leu Gly Arg Leu Arg Phe Thr Lys Pro
CAG TCT CTG ACC AGG TGG TCT GAT ATT TGG ACT 330
Gln Ala Thr Lys Tyr Ala Asn Ser Cys Cys Gln
GCC ACA AAA TAT GCA AAT TCT TGC TGT CAG AAC 363
Ser Leu Thr Arg Trp Ser Asp Ile Trp Thr Asn
ATA GAT CAT AGT TTT CCA GGC TTC CAT GGA TCA 396
Ile Asp His Ser Phe Pro Gly Phe His Gly Ser
GAG ATG TGG AAC CCA AAC ACT GAC CTC AGT GAA 429
Glu Met Trp Asn Pro Asn Thr Asp Leu Ser Glu
GAC TGT TTA TAT CTA AAT GTA TGG ATT CCA GCA 462
Asp Cys Leu Tyr Leu Asn Val Trp Ile Pro Ala
CCT AAA CCA AAA AAT GCC ACT GTA TTG ATA TGG 495
Pro Lys Pro Lys Asn Ala Thr Val Leu Ile Trp
ATT TAT GGT GGT GGT TTT CAA ACT GGA ACA TCA 528
Ile Tyr Gly Gly Gly Phe Gln Thr Gly Thr Ser
TCT TTA CAT GTT TAT GAT GGC AAG TTT CTG GCT 561
Ser Leu His Val Tyr Asp Gly Lys Phe Leu Ala
CGG GTT GAA AGA GTT ATT GTA GTG TCA ATG AAC 594
Arg Val Glu Arg Val Ile Val Val Ser Met Asn
TAT AGG GTG GGT GCC CTA GGA TTC TTA GCT TTG 627
Tyr Arg Val Gly Ala Leu Gly Phe Leu Ala Leu
CCA GGA AAT CCT GAG GCT CCA GGG AAC ATG GGT 660
Pro Gly Asn Pro Glu Ala Pro Gly Asn Met Gly
TTA TTT GAT CAA CAG TTG GCT CTT CAG TGG GTT 693
Leu Phe Asp Gln Gln Leu Ala Leu Gln Trp Val
CAA AAA AAT ATA GCA GCC TTT GGT GGA AAT CCT 726
Gln Lys Asn Ile Ala Ala Phe Gly Gly Asn Pro
AAA AGT GTA ACT CTC TTT GGA GAA AGT GCA GGA 759
Lys Ser Val Thr Leu Phe Gly Glu Ser Ala Gly
GCA GC                                      765
Ala Ala
```

The FBChEI2 clone was further employed as a probe, to search for similar sequences in cDNA libraries derived from other tissue origins.

Several such clones were thus isolated from a fetal liver cDNA library and from a primary glioblastoma cDNA library, cloned in λgt10 phages.

The full-length sequence of the fetal human cholinesterase was deduced from these clones, as presented in the following FChE scheme.

30 60

CCG TCG ACC CCT GCA TTT CCC CGA AGT ATT TCC CCG AAC TAT TAC ATG ATT TTC ACT CCT

Pro Ser Thr Pro Ala Phe Pro Arg Ser Ile Ser Pro Asn Tyr Tyr Met Ile Phe Thr Pro

90 120

TGC AAA GTT TGC CAT CTT TGT TGC AGA GAA TCG GAA ATC AAT ATG CAT AGC AAA GTC ACA

Cys Lys Val Cys His Leu Cys Cys Arg Glu Ser Glu Ile Asn Met His Ser Lys Val Thr

150 180

ATC ATA TGC ATC AGA TTT CTC TTT TGG TTT GTT TTG CTC TGC ATG CTT ATT GGG AAG TCA

Ile Ile Cys Ile Arg Phe Leu Phe Trp Phe Val Leu Leu Cys Met Leu Ile Gly Lys Ser

210 240

CAT ACT GAA GAT GAC ATC ATA ATT GCA ACA AAG AAT GGA AAA GTC AGA GGG ATG AAC TTG

His Thr Glu Asp Asp Ile Ile Ile Ala Thr Lys Asn Gly Lys Val Arg Gly Met Asn Leu

270 300

ACA GTT TTT GGT GGC ACG GTA ACA GCC TTT CTT GGA ATT CCC TAT GCA CAG CCA CCT CTT

Thr Val Phe Gly Gly Thr Val Thr Ala Phe Leu Gly Ile Pro Tyr Ala Gln Pro Pro Leu

330 360

GGT AGA CTT CGA TTC ACA AAG CCA CAG TCT CTG ACC AGG TGG TCT GAT ATT TGG ACT GCC

Gly Arg Leu Arg Phe Thr Lys Pro Gln Ser Leu Thr Arg Trp Ser Asp Ile Trp Thr Ala

390 420

ACA AAA TAT GCA AAT TCT TGC TGT CAG AAC ATA GAT CAT AGT TTT CCA GGC TTC CAT GGA

Thr Lys Tyr Ala Asn Ser Cys Cys Gln Asn Ile Asp His Ser Phe Pro Gly Phe His Gly

450 480

TCA GAG ATG TGG AAC CCA AAC ACT GAC CTC AGT GAA GAC TGT TTA TAT CTA AAT GTA TGG

Ser Glu Met Trp Asn Pro Asn Thr Asp Leu Ser Glu Asp Cys Leu Tyr Leu Asn Val Trp

510 540

ATT CCA GCA CCT AAA CCA AAA AAT GCC ACT GTA TTG ATA TGG ATT TAT GGT GGT GGT TTT

Ile Pro Ala Pro Lys Pro Lys Asn Ala Thr Val Leu Ile Trp Ile Tyr Gly Gly Gly Phe

570 600

CAA ACT GGA ACA TCA TCT TTA CAT GTT TAT GAT GGC AAG TTT CTG GCT CGG GTT GAA AGA

Gln Thr Gly Thr Ser Ser Leu His Val Tyr Asp Gly Lys Phe Leu Ala Arg Val Glu Arg

630 660

GTT ATT GTA GTG TCA ATG AAC TAT AGG GTG GGT GCC CTA GGA TTC TTA GCT TTG CCA GGA

Val Ile Val Val Ser Met Asn Tyr Arg Val Gly Ala Leu Gly Phe Leu Ala Leu Pro Gly

690 720

AAT CCT GAG GCT CCA GGG AAC ATG GGT TTA TTT GAT CAA CAG TTG GCT CTT CAG TGG GTT

Asn Pro Glu Ala Pro Gly Asn Met Gly Leu Phe Asp Gln Gln Leu Ala Leu Gln Trp Val

750 780

CAA AAA AAT ATA GCA GCC TTT GGT GGA AAT CCT AAA AGT GTA ACT CTC TTT GGA GAA AGT

Gln Lys Asn Ile Ala Ala Phe Gly Gly Asn Pro Lys Ser Val Thr Leu Phe Gly Glu Ser

810 840

GCA GGA GCA GCT TCA GTT AGC CTG CAT TTG CTT TCT CCT GGA AGC CAT TCA TTG TTC ACC

Ala Gly Ala Ala Ser Val Ser Leu His Leu Leu Ser Pro Gly Ser His Ser Leu Phe Thr

870 900

AGA GCC ATT CTG CAA AGT GGA TCC TTT AAT GCT CCT TGG GCG GTA ACA TCT CTT TAT GAA

Arg Ala Ile Leu Gln Ser Gly Ser Phe Asn Ala Pro Trp Ala Val Thr Ser Leu Tyr Glu

930 960
GCT AGG AAC AGA ACG TTG AAC TTA GCT AAA TTG ACT GGT TGC TCT AGA GAG AAT GAG ACT
Ala Arg Asn Arg Thr Leu Asn Leu Ala Lys Leu Thr Gly Cys Ser Arg Glu Asn Glu Thr

990 1020
GAA ATA ATC AAG TGT CTT AGA AAT AAA GAT CCC CAA GAA ATT CTT CTG AAT GAA GCA TTT
Glu Ile Ile Lys Cys Leu Arg Asn Lys Asp Pro Gln Glu Ile Leu Leu Asn Glu Ala Phe

1050 1080
GTT GTC CCC TAT GGG ACT CCT TTG TCA GTA AAC TTT GGT CCG ACC GTG GAT GGT GAT TTT
Val Val Pro Tyr Gly Thr Pro Leu Ser Val Asn Phe Gly Pro Thr Val Asp Gly Asp Phe

1110 1140
CTC ACT GAC ATG CCA GAC ATA TTA CTT GAA CTT GGA CAA TTT AAA AAA ACC CAG ATT TTG
Leu Thr Asp Met Pro Asp Ile Leu Leu Glu Leu Gly Gln Phe Lys Lys Thr Gln Ile Leu

1170 1200
GTG GGT GTT AAT AAA GAT GAA GGG ACA GCT TTT TTA GTC TAT GGT GCT CCT GGC TTC AGC
Val Gly Val Asn Lys Asp Glu Gly Thr Ala Phe Leu Val Tyr Gly Ala Pro Gly Phe Ser

1230 1260
AAA GAT AAC ATT AGT ATC ATA ACT AGA AAA GAA TTT CAA GAA GGT TTA AAA ATA TTT TTT
Lys Asp Asn Ile Ser Ile Ile Thr Arg Lys Glu Phe Gln Glu Gly Leu Lys Ile Phe Phe

1290 1320
CCA GGA GTG AGT GAG TTT GGA AAG GAA TCC ATC CTT TTT CAA TAC ACA GAC TGG GTA GAT
Pro Gly Val Ser Glu Phe Gly Lys Glu Ser Ile Leu Phe Gln Tyr Thr Asp Trp Val Asp

1350 1380
GAT CAA AGA CCT GAA AAC TAC CGT GAG GCC TTG GGT TGT ATG TTG TTG GGG ATT ATA ATT
Asp Gln Arg Pro Glu Asn Tyr Arg Glu Ala Leu Gly Cys Met Leu Leu Gly Ile Ile Ile

1410 1440
TCA TAT GCC CTG CCG TTT GAA GTT ACC AAG AAG TTT TCA GAA TGG GGA AAT AAT GCC TTT
Ser Tyr Ala Leu Pro Phe Glu Val Thr Lys Lys Phe Ser Glu Trp Gly Asn Asn Ala Phe

1470 1500
TTC TAC TAT TTT GAA CAC CGA TCC TCC AAA CTT CCG TGG CCA GAA TGG ATG GGA GTG ATG
Phe Tyr Tyr Phe Glu His Arg Ser Ser Lys Leu Pro Trp Pro Glu Trp Met Gly Val Met

1530 1560
CAT GGC TAT AAA TTG AAT TTG TCT TTG GTT TAC CTC TGG AAA GAA GAG ATA ATT ACA CAA
His Gly Tyr Lys Leu Asn Leu Ser Leu Val Tyr Leu Trp Lys Glu Glu Ile Ile Thr Gln

1590 1620
AAT CCT ATT AAA TTT AAG TAC ATC CAT AGT AAA CGG TGG GCA AAT TTT GCA AAA TAT GGG
Asn Pro Ile Lys Phe Lys Tyr Ile His Ser Lys Arg Trp Ala Asn Phe Ala Lys Tyr Gly

1650 1680
AAT CCA AAT GAG ACT CAG ACC ATT AGC ACA AGC TGG CCT GTC TTA AAA GCA CTG AAC AAA
Asn Pro Asn Glu Thr Gln Thr Ile Ser Thr Ser Trp Pro Val Leu Lys Ala Leu Asn Lys

1710 1740
ATA TCT AAC CTT GAA TAC AGA GTC AAC AAG AAT AAT GAC GAA ACT ACG TGC TCA ACA ATG
Ile Ser Asn Leu Glu Tyr Arg Val Asn Lys Asn Asn Asp Glu Thr Thr Cys Ser Thr Met

1770 1800
TCG ATT CTG GAC ATC ATT TTT TCC AAA AGT CTT GGA AAT GAC AGG AAA TAT GAT GAA GCA
Ser Ile Leu Asp Ile Ile Phe Ser Lys Ser Leu Gly Asn Asp Arg Lys Tyr Asp Glu Ala

```
                                            1830                                       1860
GAA TGG GAG TGG AAA GCA GGA TTC CAT CGC TGG AAC AAT TAC ATG ATG GAC TGG AAA AAT
Glu Trp Glu Trp Lys Ala Gly Phe His Arg Trp Asn Asn Tyr Met Met Asp Trp Lys Asn

                                            1890                                       1920
CAA TTT AAC GAT TAC ACT AGC AAG AAA GAA AGT TGT GTG GGT CTC TAA TTA ATA GAT TTA
Gln Phe Asn Asp Tyr Thr Ser Lys Lys Glu Ser Cys Val Gly Leu End

                                            1950                                       1980
CCC TTT ATA GAA CAT ATT TTC CTT TAG ATC AAG GCA AAA ATA TCA GGA GCT TTT TTA CAC

                                            2010                                       2040
ACC TAC TAA AAA AGT TAT TAT GTA GCT GAA ACA CAA ATG CCA GAA GGA TAA TAT TGT TCC

                                            2070                                       2100
TCA CAT CTT TAA CTT AGT ATT TTA CCA TGC ATT TCA AAA CCC AAA TGG CTA GAA CAT GTT

                                            2130                                       2160
TAA TTA AAT TTC ACA ATA TAA AGT TCT ACA GTT AAT TAT GTG CAT ATT AAA ACA TGG CCT

                                            2190                                       2220
GGT TCA ATT TCT TTC TTT CCT TAA TAA ATT TAA GTT TTT TCC CCC CAA AAT TAT CAG TGC

                                            2250                                       2280
TCT GCT TTT AGT CAC GTG TAT TTT CAT TAC CAC TCG TAA AAA GGT ATC TTT TTT AAA TGA

                                            2310                                       2340
AGT TAA ATA TTG AAA CAC TGT ACA CCA TAG TTT ACA ATA ATT AGT GTT TCC TAA GTT AAA

                                            2370                                       2400
ATA AGA ATT GAA TGT CAA TAA TGA GAA TAA TTA AAA TAA GCA CAG AAA ATC ACA AAA AAA

                                            2430
AAC AAA AAA AAA AAA AAA AAA AAA AAA AAA AAA AAA AAA AAA AAA
```

The FChE sequence includes the exact N-terminus, active site and C-terminus peptides published for human ψChE (Lockridge, ibid. (1984)). In Northern and Southern Gel hybridizations, it yields the same results as FBChEI2. When used as a probe to screen a genomic DNA library in Charon 4A prepared with partial Hoe-3 digestion, it hybridized with 5 positive phages.

Clones FBChEI2 and FChE currently serve for further screens of our cDNA libraries, aimed to isolate additional cholinesterase cDNA clones and determine the full amino acid sequence of other human cholinesterases.

In addition, it will be used for studies of the organization, structure and regulation of the human gene(s) coding for cholinesterases, as well as their mRNA transcripts and protein products.

When inserted into suitable expression vectors the above defined cDNA can direct the production of authentic human ChE with catalytic activity. The ChE thus produced can interact with OP-poisons and succinylcholine. Such ChE should be highly effective as antidote against the above compounds.

Eukaryotic cells containing such constructs are suited for the large scale production of catalytically active ChE-type products.

Examples:

I. Brain tissues were prepared as described (Razon, N., Soreq, H., Roth, E., Bartal, A. and Silman I. Exp. Neruol. 84 681-695 (1984)).

II. Preparation of polyadenylated RNA from eukaryotic tissue sources was effected as described (Prody, C., Zevin-Sonkin, D., Gnatt, A., Koch, R., Zisling, R., Goldberg, O. and Soreq, H., J. Neurosci. Res., in press (1986)).

III. Size fractionation of DNA and RNA by gel electrophoresis and sucrose gradient centrifugation was carried out as described (Soreq, H. et al., ibid. (1984); Soreq, H. et al., Proc. Natl. Acad. Sci. USA, 82, 1827-1831, (1985); Burmeister, M. et al., EMBO Journal 3, 1499-1505, (1984));

IV. Protein gel electrophoresis was effected by gradient polyacrylamide gel electrophoresis (5-15%), (Giveon, D., in; Molecular Biology Approach to the Neurosciences, Soreq, H., ed. Series of Methods in Neurochemistry. IBRO Handbook series, vol. 7, John Wiley and Sons, New York, pp 231-243, (1984)).

V. Injection of mRNA into Xenopus oocytes was as described by Soreq, H., et al., ibid. (1982); Soreq, H. ibid., (1984), ibid., (1985);

VI. Cholinesterase radiometric assay was carried out as described by Soreq, H., et al., ibid. (1984), Razon, N. et al., ibid. (1984), Zakut, H. et al., J. Neurochem. US, 382-389 (1985);

VII. Human genomic DNA library was generated by cloning partial Hae-3 cleavage fragments of peripheral blood DNA in Charon 4A lambda phages. (Maniatis et al., ibid. (1982), and E. Coli K-12 bacteria were from strain LE 392 ($F^-$, hsd R514($r_k$ $m_k$ ) supF58 LacY, gal K2, galT22, metBl, trpR55) and amplified by standard procedure (Maniatis et al., Molecular Cloning, a laboratory manual; Cold Spring Harbor Laboratory (1982)).

VIII. Digestion with restriction enzymes was performed in each case under the conditions recommended by the suppliers, New England Biolabs. The enzymatic reactions were carried out at least for 2 h at the appropriate temperature.

IX. Electroelution of DNA fragments from agarose gel, nick-translation of DNA and preparation of phage DNA (large scale) was as described (Soreq et al., ibid. (1985).

X. DNA sequencing by the Sanger dideoxy technique was according to Sanger. F., Science 214, 1205-1210 (1981), six percent 0.2. mm thick acrylamide gels were used (Sanger et al,m FEBS Lett. 87, 107-110 (1978), Guroff et al., Analyt. Bioch. 115, 450-457 1981).

The DNA sequences defined by the Sanger dideoxy technique were analyzed by the RUNSEQ (Sege et al., Nuc. Acid Res. 9 437-444 (1981)) program available at the WICC.

XI. Synthesis of OPSYN oligonucleotides was carried out as described (Prody et al., ibid. (1986))

XII. Strategy for the order and synthesis and screening of OPSYN Oligonucleotides.

A mixture of the following 9-mer oligonucleotides was included in the 5'terminal end of all of the OPSYN preparations:

```
PHE   Gly   Glu
                   (M=A or G, N=A, C, G or T)
AAM   CCA   CTC
              T
```

This mixture was divided into six parts, each of which was succeeded by one of the codons for serine (AGN or TCM). These were, again, divided into four mixtures each, to which we added

| | |
|---|---|
| Ala<br>CGN | Gly<br>CC |

with N being either A,C,G, or T.

XIII. End labelling of Synthetic Oligonucleotides with [$^{32}$P]ATP was according to Maniatis' handbook (1982) and as described (Prody et al., 1986).

XIV. The minimal length of the full-length FChE DNA which is necessary and sufficient for the production of catalytically active ChE in an appropriate expression system, is defined by site-directed mutagenesis of expressible partial ChE cDNA clones.

As bet out above, a DNA expression vector comprising a DNA sequence encoding a protein having human ChE activity (AChE and/or pseudo-ChE activity) can be used for the production of such proteins. The production can be effected by means of bacterial, eukaryotic cells or yeast cells.

The invention also encompasses such biologically active proteins, whenever obtained by the cultivation of such cells.

Suitable systems in which catalytically active ChE of human origin can be produced by the above described FChE cDNA sequences are thus provided. These include host systems such as bacteria, yeast or mammalian cells in culture as well as engineered constructs, composed of appropriate expression vectors and the entire or part of the above described ChE cDNA sequences.

XV. Methods for the large scale production of authentic human-originated catalytically active ChE is provided, based on the use of expression systems as in XIV, transfected with constructs in which a cDNA coding for ChE has been joined end-to-end with an expression effecting DNA in an operable manner.

The proteins having human AChE or human pseudo-ChE activity can be used as active ingredient in compositions having utility as prophylactics against poisoning by organophosphorus (OP) compounds, as antidotes against such OP poisoning or blocking by succinylcholine and for counteracting the effects of such OP or succinylcholine compounds. The effective doses of such active proteins is in the milligram range. Such compositions can contain further active ingredients, of the type used for counteracting the effects of such OP compounds.

XVI. The minimal size of the authentic human-originated active ChE that is sufficient for protection against OP agents is provided, as defined by the synthesis of such catalytically active enzyme from human ChE cDNA shortened or modified by molecular engineering techniques, reinserted into expression vector constructs and expressed in a suitable host system to yield a biologically active protein which interacts efficiently with OP agents.

XVII. The protein domain that is actively involved in OP-binding is provided as in Table I, II and II.

In the following, Table I defines a protein sequence that is encoded by the full-length FChE cDNA sequence and includes the complete primary sequence of human pseudo ChE, preceded by a signal peptide and an upstream peptide. Table II defines a pseudo ChE protein sequence initiating with methionine and including the signal peptide alone, and Table III defines the mature pseudo ChE protein sequence, devoid of the signal and preceding peptides.

Table 1

10 20
Pro Ser Thr Pro Ala Phe Pro Arg Ser Ile Ser Pro Asn Tyr Tyr Met Ile Phe Thr Pro

30 40
Cys Lys Val Cys His Leu Cys Cys Arg Glu Ser Glu Ile Asn Met His Ser Lys Val Thr

50 60
Ile Ile Cys Ile Arg Phe Leu Phe Trp Phe Val Leu Leu Cys Met Leu Ile Gly Lys Ser

70 80
His Thr Glu Asp Asp Ile Ile Ile Ala Thr Lys Asn Gly Lys Val Arg Gly Met Asn Leu

90 100
Thr Val Phe Gly Gly Thr Val Thr Ala Phe Leu Gly Ile Pro Tyr Ala Gln Pro Pro Leu

110 120
Gly Arg Leu Arg Phe Thr Lys Pro Gln Ser Leu Thr Arg Trp Ser Asp Ile Trp Thr Ala

130 140
Thr Lys Tyr Ala Asn Ser Cys Cys Gln Asn Ile Asp His Ser Phe Pro Gly Phe His Gly

150 160
Ser Glu Met Trp Asn Pro Asn Thr Asp Leu Ser Glu Asp Cys Leu Tyr Leu Asn Val Trp

170 180
Ile Pro Ala Pro Lys Pro Lys Asn Ala Thr Val Leu Ile Trp Ile Tyr Gly Gly Gly Phe

190 200
Gln Thr Gly Thr Ser Ser Leu His Val Tyr Asp Gly Lys Phe Leu Ala Arg Val Glu Arg

210 220
Val Ile Val Val Ser Met Asn Tyr Arg Val Gly Ala Leu Gly Phe Leu Ala Leu Pro Gly

230 240
Asn Pro Glu Ala Pro Gly Asn Met Gly Leu Phe Asp Gln Gln Leu Ala Leu Gln Trp Val

250 260
Gln Lys Asn Ile Ala Ala Phe Gly Gly Asn Pro Lys Ser Val Thr Leu Phe Gly Glu Ser

270 280
Ala Gly Ala Ala Ser Val Ser Leu His Leu Leu Ser Pro Gly Ser His Ser Leu Phe Thr

290 300
Arg Ala Ile Leu Gln Ser Gly Ser Phe Asn Ala Pro Trp Ala Val Thr Ser Leu Tyr Glu

310 320
Ala Arg Asn Arg Thr Leu Asn Leu Ala Lys Leu Thr Gly Cys Ser Arg Glu Asn Glu Thr

330 340
Glu Ile Ile Lys Cys Leu Arg Asn Lys Asp Pro Gln Glu Ile Leu Leu Asn Glu Ala Phe

350 360
Val Val Pro Tyr Gly Thr Pro Leu Ser Val Asn Phe Gly Pro Thr Val Asp Gly Asp Phe

370 380
Leu Thr Asp Met Pro Asp Ile Leu Leu Glu Leu Gly Gln Phe Lys Lys Thr Gln Ile Leu

390 400
Val Gly Val Asn Lys Asp Glu Gly Thr Ala Phe Leu Val Tyr Gly Ala Pro Gly Phe Ser

410 420
Lys Asp Asn Ile Ser Ile Ile Thr Arg Lys Glu Phe Gln Glu Gly Leu Lys Ile Phe Phe

/continued

continuation of Table 1

430
Pro Gly Val Ser Glu Phe Gly Lys Glu Ser Ile Leu Phe Gln Tyr Thr Asp Trp Val Asp

450 460
Asp Gln Arg Pro Glu Asn Tyr Arg Glu Ala Leu Gly Cys Met Leu Leu Gly Ile Ile Ile

470 480
Ser Tyr Ala Leu Pro Phe Glu Val Thr Lys Lys Phe Ser Glu Trp Gly Asn Asn Ala Phe

490 500
Phe Tyr Tyr Phe Glu His Arg Ser Ser Lys Leu Pro Trp Pro Glu Trp Met Gly Val Met

510 520
His Gly Tyr Lys Leu Asn Leu Ser Leu Val Tyr Leu Trp Lys Glu Glu Ile Ile Thr Gln

530 540
Asn Pro Ile Lys Phe Lys Tyr Ile His Ser Lys Arg Trp Ala Asn Phe Ala Lys Tyr Gly

550 560
Asn Pro Asn Glu Thr Gln Thr Ile Ser Thr Ser Trp Pro Val Leu Lys Ala Leu Asn Lys

570 580
Ile Ser Asn Leu Glu Tyr Arg Val Asn Lys Asn Asn Asp Glu Thr Thr Cys Ser Thr Met

590 600
Ser Ile Leu Asp Ile Ile Phe Ser Lys Ser Leu Gly Asn Asp Arg Lys Tyr Asp Glu Ala

610 620
Glu Trp Glu Trp Lys Ala Gly Phe His Arg Trp Asn Asn Tyr Met Met Asp Trp Lys Asn

630
Gln Phe Asn Asp Tyr Thr Ser Lys Lys Glu Ser Cys Val Gly Leu

che-sp

Table II

10 20
Met His Ser Lys Val Thr Ile Ile Cys Ile Arg Phe Leu Phe Trp Phe Val Leu Leu Cys

30 40
Met Leu Ile Gly Lys Ser His Thr Glu Asp Asp Ile Ile Ile Ala Thr Lys Asn Gly Lys

50 60
Val Arg Gly Met Asn Leu Thr Val Phe Gly Gly Thr Val Thr Ala Phe Leu Gly Ile Pro

70 80
Tyr Ala Gln Pro Pro Leu Gly Arg Leu Arg Phe Thr Lys Pro Gln Ser Leu Thr Arg Trp

90 100
Ser Asp Ile Trp Thr Ala Thr Lys Tyr Ala Asn Ser Cys Cys Gln Asn Ile Asp His Ser

110 120
Phe Pro Gly Phe His Gly Ser Glu Met Trp Asn Pro Asn Thr Asp Leu Ser Glu Asp Cys

130 140
Leu Tyr Leu Asn Val Trp Ile Pro Ala Pro Lys Pro Lys Asn Ala Thr Val Leu Ile Trp

150 160
Ile Tyr Gly Gly Gly Phe Gln Thr Gly Thr Ser Ser Leu His Val Tyr Asp Gly Lys Phe

170 180
Leu Ala Arg Val Glu Arg Val Ile Val Val Ser Met Asn Tyr Arg Val Gly Ala Leu Gly

190 200
Phe Leu Ala Leu Pro Gly Asn Pro Glu Ala Pro Gly Asn Met Gly Leu Phe Asp Gln Gln

210 220
Leu Ala Leu Gln Trp Val Gln Lys Asn Ile Ala Ala Phe Gly Gly Asn Pro Lys Ser Val

230 240
Thr Leu Phe Gly Glu Ser Ala Gly Ala Ala Ser Val Ser Leu His Leu Leu Ser Pro Gly

250 260
Ser His Ser Leu Phe Thr Arg Ala Ile Leu Gln Ser Gly Ser Phe Asn Ala Pro Trp Ala

270 280
Val Thr Ser Leu Tyr Glu Ala Arg Asn Arg Thr Leu Asn Leu Ala Lys Leu Thr Gly Cys

290 300
Ser Arg Glu Asn Glu Thr Glu Ile Ile Lys Cys Leu Arg Asn Lys Asp Pro Gln Glu Ile

310 320
Leu Leu Asn Glu Ala Phe Val Val Pro Tyr Gly Thr Pro Leu Ser Val Asn Phe Gly Pro

330 340
Thr Val Asp Gly Asp Phe Leu Thr Asp Met Pro Asp Ile Leu Leu Glu Leu Gly Gln Phe

350 360
Lys Lys Thr Gln Ile Leu Val Gly Val Asn Lys Asp Glu Gly Thr Ala Phe Leu Val Tyr

370 380
Gly Ala Pro Gly Phe Ser Lys Asp Asn Ile Ser Ile Ile Thr Arg Lys Glu Phe Gln Glu

390 400
Gly Leu Lys Ile Phe Phe Pro Gly Val Ser Glu Phe Gly Lys Glu Ser Ile Leu Phe Gln

410 420
Tyr Thr Asp Trp Val Asp Asp Gln Arg Pro Glu Asn Tyr Arg Glu Ala Leu Gly Cys Met

/ continued

cont. of Table II

430 440
Leu Leu Gly Ile Ile Ile Ser Tyr Ala Leu Pro Phe Glu Val Thr Lys Lys Phe Ser Glu

450 460
Trp Gly Asn Asn Ala Phe Phe Tyr Tyr Phe Glu His Arg Ser Ser Lys Leu Pro Trp Pro

470 480
Glu Trp Met Gly Val Met His Gly Tyr Lys Leu Asn Leu Ser Leu Val Tyr Leu Trp Lys

490 500
Glu Glu Ile Ile Thr Gln Asn Pro Ile Lys Phe Lys Tyr Ile His Ser Lys Arg Trp Ala

510 520
Asn Phe Ala Lys Tyr Gly Asn Pro Asn Glu Thr Gln Thr Ile Ser Thr Ser Trp Pro Val

530 540
Leu Lys Ala Leu Asn Lys Ile Ser Asn Leu Glu Tyr Arg Val Asn Lys Asn Asn Asp Glu

550 560
Thr Thr Cys Ser Thr Met Ser Ile Leu Asp Ile Ile Phe Ser Lys Ser Leu Gly Asn Asp

570 580
Arg Lys Tyr Asp Glu Ala Glu Trp Glu Trp Lys Ala Gly Phe His Arg Trp Asn Asn Tyr

590 600
Met Met Asp Trp Lys Asn Gln Phe Asn Asp Tyr Thr Ser Lys Lys Glu Ser Cys Val Gly

Leu

Table III

| | | | | | | | | | |
|---|---|---|---|---|---|---|---|---|---|
| | | | | | | | | | 10 | | | | | | | | | | 20 |
| Glu | Asp | Asp | Ile | Ile | Ile | Ala | Thr | Lys | Asn | Gly | Lys | Val | Arg | Gly | Met | Asn | Leu | Thr | Val |
| | | | | | | | | | 30 | | | | | | | | | | 40 |
| Phe | Gly | Gly | Thr | Val | Thr | Ala | Phe | Leu | Gly | Ile | Pro | Tyr | Ala | Gln | Pro | Pro | Leu | Gly | Arg |
| | | | | | | | | | 50 | | | | | | | | | | 60 |
| Leu | Arg | Phe | Thr | Lys | Pro | Gln | Ser | Leu | Thr | Arg | Trp | Ser | Asp | Ile | Trp | Thr | Ala | Thr | Lys |
| | | | | | | | | | 70 | | | | | | | | | | 80 |
| Tyr | Ala | Asn | Ser | Cys | Cys | Gln | Asn | Ile | Asp | His | Ser | Phe | Pro | Gly | Phe | His | Gly | Ser | Glu |
| | | | | | | | | | 90 | | | | | | | | | | 100 |
| Met | Trp | Asn | Pro | Asn | Thr | Asp | Leu | Ser | Glu | Asp | Cys | Leu | Tyr | Leu | Asn | Val | Trp | Ile | Pro |
| | | | | | | | | | 110 | | | | | | | | | | 120 |
| Ala | Pro | Lys | Pro | Lys | Asn | Ala | Thr | Val | Leu | Ile | Trp | Ile | Tyr | Gly | Gly | Gly | Phe | Gln | Thr |
| | | | | | | | | | 130 | | | | | | | | | | 140 |
| Gly | Thr | Ser | Ser | Leu | His | Val | Tyr | Asp | Gly | Lys | Phe | Leu | Ala | Arg | Val | Glu | Arg | Val | Ile |
| | | | | | | | | | 150 | | | | | | | | | | 160 |
| Val | Val | Ser | Met | Asn | Tyr | Arg | Val | Gly | Ala | Leu | Gly | Phe | Leu | Ala | Leu | Pro | Gly | Asn | Pro |
| | | | | | | | | | 170 | | | | | | | | | | 180 |
| Glu | Ala | Pro | Gly | Asn | Met | Gly | Leu | Phe | Asp | Gln | Gln | Leu | Ala | Leu | Gln | Trp | Val | Gln | Lys |
| | | | | | | | | | 190 | | | | | | | | | | 200 |
| Asn | Ile | Ala | Ala | Phe | Gly | Gly | Asn | Pro | Lys | Ser | Val | Thr | Leu | Phe | Gly | Glu | Ser | Ala | Gly |
| | | | | | | | | | 210 | | | | | | | | | | 220 |
| Ala | Ala | Ser | Val | Ser | Leu | His | Leu | Leu | Ser | Pro | Gly | Ser | His | Ser | Leu | Phe | Thr | Arg | Ala |
| | | | | | | | | | 230 | | | | | | | | | | 240 |
| Ile | Leu | Gln | Ser | Gly | Ser | Phe | Asn | Ala | Pro | Trp | Ala | Val | Thr | Ser | Leu | Tyr | Glu | Ala | Arg |
| | | | | | | | | | 250 | | | | | | | | | | 260 |
| Asn | Arg | Thr | Leu | Asn | Leu | Ala | Lys | Leu | Thr | Gly | Cys | Ser | Arg | Glu | Asn | Glu | Thr | Glu | Ile |
| | | | | | | | | | 270 | | | | | | | | | | 280 |
| Ile | Lys | Cys | Leu | Arg | Asn | Lys | Asp | Pro | Gln | Glu | Ile | Leu | Leu | Asn | Glu | Ala | Phe | Val | Val |
| | | | | | | | | | 290 | | | | | | | | | | 300 |
| Pro | Tyr | Gly | Thr | Pro | Leu | Ser | Val | Asn | Phe | Gly | Pro | Thr | Val | Asp | Gly | Asp | Phe | Leu | Thr |
| | | | | | | | | | 310 | | | | | | | | | | 320 |
| Asp | Met | Pro | Asp | Ile | Leu | Leu | Glu | Leu | Gly | Gln | Phe | Lys | Lys | Thr | Gln | Ile | Leu | Val | Gly |
| | | | | | | | | | 330 | | | | | | | | | | 340 |
| Val | Asn | Lys | Asp | Glu | Gly | Thr | Ala | Phe | Leu | Val | Tyr | Gly | Ala | Pro | Gly | Phe | Ser | Lys | Asp |
| | | | | | | | | | 350 | | | | | | | | | | 360 |
| Asn | Ile | Ser | Ile | Ile | Thr | Arg | Lys | Glu | Phe | Gln | Glu | Gly | Leu | Lys | Ile | Phe | Phe | Pro | Gly |
| | | | | | | | | | 370 | | | | | | | | | | 380 |
| Val | Ser | Glu | Phe | Gly | Lys | Glu | Ser | Ile | Leu | Phe | Gln | Tyr | Thr | Asp | Trp | Val | Asp | Asp | Gln |
| | | | | | | | | | 390 | | | | | | | | | | 400 |
| Arg | Pro | Glu | Asn | Tyr | Arg | Glu | Ala | Leu | Gly | Cys | Met | Leu | Leu | Gly | Ile | Ile | Ile | Ser | Tyr |
| | | | | | | | | | 410 | | | | | | | | | | 420 |
| Ala | Leu | Pro | Phe | Glu | Val | Thr | Lys | Lys | Phe | Ser | Glu | Trp | Gly | Asn | Asn | Ala | Phe | Phe | Tyr |

/continued

continuation of Table III

| | | | | | | | | | | | | | | | | | | | |
|---|---|---|---|---|---|---|---|---|---|---|---|---|---|---|---|---|---|---|---|
| | | | | | | | | | 430 | | | | | | | | | | 440 |
| Tyr | Phe | Glu | His | Arg | Ser | Ser | Lys | Leu | Pro | Trp | Pro | Glu | Trp | Met | Gly | Val | Met | His | Gly |
| | | | | | | | | | 450 | | | | | | | | | | 460 |
| Tyr | Lys | Leu | Asn | Leu | Ser | Leu | Val | Tyr | Leu | Trp | Lys | Glu | Glu | Ile | Ile | Thr | Gln | Asn | Pro |
| | | | | | | | | | 470 | | | | | | | | | | 480 |
| Ile | Lys | Phe | Lys | Tyr | Ile | His | Ser | Lys | Arg | Trp | Ala | Asn | Phe | Ala | Lys | Tyr | Gly | Asn | Pro |
| | | | | | | | | | 490 | | | | | | | | | | 500 |
| Asn | Glu | Thr | Gln | Thr | Ile | Ser | Thr | Ser | Trp | Pro | Val | Leu | Lys | Ala | Leu | Asn | Lys | Ile | Ser |
| | | | | | | | | | 510 | | | | | | | | | | 520 |
| Asn | Leu | Glu | Tyr | Arg | Val | Asn | Lys | Asn | Asn | Asp | Glu | Thr | Thr | Cys | Ser | Thr | Met | Ser | Ile |
| | | | | | | | | | 530 | | | | | | | | | | 540 |
| Leu | Asp | Ile | Ile | Phe | Ser | Lys | Ser | Leu | Gly | Asn | Asp | Arg | Lys | Tyr | Asp | Glu | Ala | Glu | Trp |
| | | | | | | | | | 550 | | | | | | | | | | 560 |
| Glu | Trp | Lys | Ala | Gly | Phe | His | Arg | Trp | Asn | Asn | Tyr | Met | Met | Asp | Trp | Lys | Asn | Gln | Phe |
| | | | | | | | | | 570 | | | | | | | | | | |
| Asn | Asp | Tyr | Thr | Ser | Lys | Lys | Glu | Ser | Cys | Val | Gly | Leu | | | | | | | |

## Claims

**Claims for the following Contracting States : BE, CH, DE, FR, GB, IT, LI, NL, SE**

**1.** A DNA sequence of the formula

```
                                             30                                                60
CCG TCG ACC CCT GCA TTT CCC CGA AGT ATT TCC CCG AAC TAT TAC ATG ATT TTC ACT CCT
Pro Ser Thr Pro Ala Phe Pro Arg Ser Ile Ser Pro Asn Tyr Tyr Met Ile Phe Thr Pro

                                             90                                               120
TGC AAA GTT TGC CAT CTT TGT TGC AGA GAA TCG GAA ATC AAT ATG CAT AGC AAA GTC ACA
Cys Lys Val Cys His Leu Cys Cys Arg Glu Ser Glu Ile Asn Met His Ser Lys Val Thr

                                            150                                               180
ATC ATA TGC ATC AGA TTT CTC TTT TGG TTT GTT TTG CTC TGC ATG CTT ATT GGG AAG TCA
Ile Ile Cys Ile Arg Phe Leu Phe Trp Phe Val Leu Leu Cys Met Leu Ile Gly Lys Ser

                                            210                                               240
CAT ACT GAA GAT GAC ATC ATA ATT GCA ACA AAG AAT GGA AAA GTC AGA GGG ATG AAC TTG
His Thr Glu Aso Asp Ile Ile Ile Ala Thr Lys Asn Gly Lys Val Arg Gly Met Asn Leu

                                            270                                               300
ACA GTT TTT GGT GGC ACG GTA ACA GCC TTT CTT GGA ATT CCC TAT GCA CAG CCA CCT CTT
Thr Val Phe Gly Gly Thr Val Thr Ala Phe Leu Gly Ile Pro Tyr Ala Gln Pro Pro Leu

                                            330                                               360
GGT AGA CTT CGA TTC ACA AAG CCA CAG TCT CTG ACC AGG TGG TCT GAT ATT TGG ACT GCC
Gly Arg Leu Arg Phe Thr Lys Pro Gln Ser Leu Thr Arg Trp Ser Asp Ile Trp Thr Ala

                                            390                                               420
ACA AAA TAT GCA AAT TCT TGC TGT CAG AAC ATA GAT CAT AGT TTT CCA GGC TTC CAT GGA
Thr Lys Tyr Ala Asn Ser Cys Cys Gln Asn Ile Asp His Ser Phe Pro Gly Phe His Gly

                                            450                                               480
TCA GAG ATG TGG AAC CCA AAC ACT GAC CTC AGT GAA GAC TGT TTA TAT CTA AAT GTA TGG
Ser Glu Met Trp Asn Pro Asn Thr Asp Leu Ser Glu Asp Cys Leu Tyr Leu Asn Val Trp

                                            510                                               540
ATT CCA GCA CCT AAA CCA AAA AAT GCC ACT GTA TTG ATA TGG ATT TAT GGT GGT GGT TTT
Ile Pro Ala Pro Lys Pro Lys Asn Ala Thr Val Leu Ile Trp Ile Tyr Gly Gly Gly Phe

                                            570                                               600
CAA ACT GGA ACA TCA TCT TTA CAT GTT TAT GAT GGC AAG TTT CTG GCT CGG GTT GAA AGA
Gln Thr Gly Thr Ser Ser Leu His Val Tyr Asp Gly Lys Phe Leu Ala Arg Val Glu Arg

                                            630                                               660
GTT ATT GTA GTG TCA ATG AAC TAT AGG GTG GGT GCC CTA GGA TTC TTA GCT TTG CCA GGA
Val Ile Val Val Ser Met Asn Tyr Arg Val Gly Ala Leu Gly Phe Leu Ala Leu Pro Gly

                                            690                                               720
AAT CCT GAG GCT CCA GGG AAC ATG GGT TTA TTT GAT CAA CAG TTG GCT CTT CAG TGG GTT
Asn Pro Glu Ala Pro Gly Asn Met Gly Leu Phe Asp Gln Gln Leu Ala Leu Gln Trp Val

                                            750                                               780
CAA AAA AAT ATA GCA GCC TTT GGT GGA AAT CCT AAA AGT GTA ACT CTC TTT GGA GAA AGT
Gln Lys Asn Ile Ala Ala Phe Gly Gly Asn Pro Lys Ser Val Thr Leu Phe Gly Glu Ser

                                            810                                               840
GCA GGA GCA GCT TCA GTT AGC CTG CAT TTG CTT TCT CCT GGA AGC CAT TCA TTG TTC ACC
Ala Gly Ala Ala Ser Val Ser Leu His Leu Leu Ser Pro Gly Ser His Ser Leu Phe Thr

                                            870                                               900
AGA GCC ATT CTG CAA AGT GGA TCC TTT AAT GCT CCT TGG GCG GTA ACA TCT CTT TAT GAA
Arg Ala Ile Leu Gln Ser Gly Ser Phe Asn Ala Pro Trp Ala Val Thr Ser Leu Tyr Glu
```

```
                                                 930                                            960
GCT AGG AAC AGA ACG TTG AAC TTA GCT AAA TTG ACT GGT TGC TCT AGA GAG AAT GAG ACT
Ala Arg Asn Arg Thr Leu Asn Leu Ala Lys Leu Thr Gly Cys Ser Arg Glu Asn Glu Thr

                                                 990                                           1020
GAA ATA ATC AAG TGT CTT AGA AAT AAA GAT CCC CAA GAA ATT CTT CTG AAT GAA GCA TTT
Glu Ile Ile Lys Cys Leu Arg Asn Lys Asp Pro Gln Glu Ile Leu Leu Asn Glu Ala Phe

                                                1050                                           1080
GTT GTC CCC TAT GGG ACT CCT TTG TCA GTA AAC TTT GGT CCG ACC GTG GAT GGT GAT TTT
Val Val Pro Tyr Gly Thr Pro Leu Ser Val Asn Phe Gly Pro Thr Val Asp Gly Asp Phe

                                                1110                                           1140
CTC ACT GAC ATG CCA GAC ATA TTA CTT GAA CTT GGA CAA TTT AAA AAA ACC CAG ATT TTG
Leu Thr Asp Met Pro Asp Ile Leu Leu Glu Leu Gly Gln Phe Lys Lys Thr Gln Ile Leu

                                                1170                                           1200
GTG GGT GTT AAT AAA GAT GAA GGG ACA GCT TTT TTA GTC TAT GGT GCT CCT GGC TTC AGC
Val Gly Val Asn Lys Asp Glu Gly Thr Ala Phe Leu Val Tyr Gly Ala Pro Gly Phe Ser

                                                1230                                           1260
AAA GAT AAC ATT AGT ATC ATA ACT AGA AAA GAA TTT CAA GAA GGT TTA AAA ATA TTT TTT
Lys Asp Asn Ile Ser Ile Ile Thr Arg Lys Glu Phe Gln Glu Gly Leu Lys Ile Phe Phe

                                                1290                                           1320
CCA GGA GTG AGT GAG TTT GGA AAG GAA TCC ATC CTT TTT CAA TAC ACA GAC TGG GTA GAT
Pro Gly Val Ser Glu Phe Gly Lys Glu Ser Ile Leu Phe Gln Tyr Thr Asp Trp Val Asp

                                                1350                                           1380
GAT CAA AGA CCT GAA AAC TAC CGT GAG GCC TTG GGT TGT ATG TTG TTG GGG ATT ATA ATT
Asp Gln Arg Pro Glu Asn Tyr Arg Glu Ala Leu Gly Cys Met Leu Leu Gly Ile Ile Ile

                                                1410                                           1440
TCA TAT GCC CTG CCG TTT GAA GTT ACC AAG AAG TTT TCA GAA TGG GGA AAT AAT GCC TTT
Ser Tyr Ala Leu Pro Phe Glu Val Thr Lys Lys Phe Ser Glu Trp Gly Asn Asn Ala Phe

                                                1470                                           1500
TTC TAC TAT TTT GAA CAC CGA TCC TCC AAA CTT CCG TGG CCA GAA TGG ATG GGA GTG ATG
Phe Tyr Tyr Phe Glu His Arg Ser Ser Lys Leu Pro Trp Pro Glu Trp Met Gly Val Met

                                                1530                                           1560
CAT GGC TAT AAA TTG AAT TTG TCT TTG GTT TAC CTC TGG AAA GAA GAG ATA ATT ACA CAA
His Gly Tyr Lys Leu Asn Leu Ser Leu Val Tyr Leu Trp Lys Glu Glu Ile Ile Thr Gln

                                                1590                                           1620
AAT CCT ATT AAA TTT AAG TAC ATC CAT AGT AAA CGG TGG GCA AAT TTT GCA AAA TAT GGG
Asn Pro Ile Lys Phe Lys Tyr Ile His Ser Lys Arg Trp Ala Asn Phe Ala Lys Tyr Gly

                                                1650                                           1680
AAT CCA AAT GAG ACT CAG ACC ATT AGC ACA AGC TGG CCT GTC TTA AAA GCA CTG AAC AAA
Asn Pro Asn Glu Thr Gln Thr Ile Ser Thr Ser Trp Pro Val Leu Lys Ala Leu Asn Lys

                                                1710                                           1740
ATA TCT AAC CTT GAA TAC AGA GTC AAC AAG AAT AAT GAC GAA ACT ACG TGC TCA ACA ATG
Ile Ser Asn Leu Glu Tyr Arg Val Asn Lys Asn Asn Asp Glu Thr Thr Cys Ser Thr Met

                                                1770                                           1800
TCG ATT CTG GAC ATC ATT TTT TCC AAA AGT CTT GGA AAT GAC AGG AAA TAT GAT GAA GCA
Ser Ile Leu Asp Ile Ile Phe Ser Lys Ser Leu Gly Asn Asp Arg Lys Tyr Asp Glu Ala
```

```
                                            1830                                    1860
GAA TGG GAG TGG AAA GCA GGA TTC CAT CGC TGG AAC AAT TAC ATG ATG GAC TGG AAA AAT
Glu Trp Glu Trp Lys Ala Gly Phe His Arg Trp Asn Asn Tyr Met Met Asp Trp Lys Asn

                                            1890                                    1920
CAA TTT AAC GAT TAC ACT AGC AAG AAA GAA AGT TGT GTG GGT CTC TAA TTA ATA GAT TTA
Gln Phe Asn Asp Tyr Thr Ser Lys Lys Glu Ser Cys Val Gly Leu End

                                            1950                                    1980
CCC TTT ATA GAA CAT ATT TTC CTT TAG ATC AAG GCA AAA ATA TCA GGA GCT TTT TTA CAC

                                            2010                                    2040
ACC TAC TAA AAA AGT TAT TAT GTA GCT GAA ACA CAA ATG CCA GAA GGA TAA TAT TGT TCC

                                            2070                                    2100
TCA CAT CTT TAA CTT AGT ATT TTA CCA TGC ATT TCA AAA CCC AAA TGG CTA GAA CAT GTT

                                            2130                                    2160
TAA TTA AAT TTC ACA ATA TAA AGT TCT ACA GTT AAT TAT GTG CAT ATT AAA ACA TGG CCT

                                            2190                                    2220
GGT TCA ATT TCT TTC TTT CCT TAA TAA ATT TAA GTT TTT TCC CCC CAA AAT TAT CAG TGC

                                            2250                                    2280
TCT GCT TTT AGT CAC GTG TAT TTT CAT TAC CAC TCG TAA AAA GGT ATC TTT TTT AAA TGA

                                            2310                                    2340
AGT TAA ATA TTG AAA CAC TGT ACA CCA TAG TTT ACA ATA ATT AGT GTT TCC TAA GTT AAA

                                            2370                                    2400
ATA AGA ATT GAA TGT CAA TAA TGA GAA TAA TTA AAA TAA GCA CAG AAA ATC ACA AAA AAA

                                            2430
AAC AAA AAA AAA AAA AAA AAA AAA AAA AAA AAA AAA AAA AAA AAA
```

or part of the sequence encoding a peptide having human pseudocholinesterase activity or operably linked to an expression vector adapted to encode a cholinesterase-like peptide interacting with antibodies to human acetylcholinesterase.

**2.** The DNA sequence of claim 1 encoding a polypeptide having pseudocholinesterase activity.

**3.** A DNA sequence according to claim 1 of the formula

```
ATT TCC CCG AAG TAT TAC ATG ATT TTC ACT CCT  33
Ile Ser Pro Lys Tyr Tyr Met Ile Phe Thr Pro
TGC AAA CTT TGC CAT CTT TGT TGC AGA GAA TCG  66
Cys Lys Leu Cys His Leu Cys Cys Arg Glu Ser
GAA ATC AAT ATG CAT AGC AAA GTC ACA ATC ATA  99
Glu Ile Asn Met His Ser Lys Val Thr Ile Ile
TGC ATC AGA TTT CTC TTT TGG TTT CTT TTG CTC 132
Cys Ile Arg Phe Leu Phe Trp Phe Leu Leu Leu
TGC ATG CTT ATT GGG AAG TCA CAT ACT GAA GAT 165
Cys Met Leu Ile Gly Lys Ser His Thr Glu Asp
GAC ATC ATA ATT GCA ACA AAG AAT GGA AAA GTC 198
Asp Ile Ile Ile Ala Thr Lys Asn Gly Lys Val
AGA GGG ATG AAC TTG ACA GTT TTT GGT GGC ACG 231
Arg Gly Met Asn Leu Thr Val Phe Gly Gly Thr
GTA ACA GCC TTT CTT GGA ATT CCC TAT GCA CAG 264
Val Thr Ala Phe Leu Gly Ile Pro Tyr Ala Gln
CCA CCT CTT GGT AGA CTT CGA TTC ACA AAG CCA 297
Pro Pro Leu Gly Arg Leu Arg Phe Thr Lys Pro
CAG TCT CTG ACC AGG TGG TCT GAT ATT TGG ACT 330
Gln Ala Thr Lys Tyr Ala Asn Ser Cys Cys Gln
GCC ACA AAA TAT GCA AAT TCT TGC TGT CAG AAC 363
Ser Leu Thr Arg Trp Ser Asp Ile Trp Thr Asn
ATA GAT CAT AGT TTT CCA GGC TTC CAT GGA TCA 396
Ile Asp His Ser Phe Pro Gly Phe His Gly Ser
GAG ATG TGG AAC CCA AAC ACT GAC CTC AGT GAA 429
Glu Met Trp Asn Pro Asn Thr Asp Leu Ser Glu
GAC TGT TTA TAT CTA AAT GTA TGG ATT CCA GCA 462
Asp Cys Leu Tyr Leu Asn Val Trp Ile Pro Ala
CCT AAA CCA AAA AAT GCC ACT GTA TTG ATA TGG 495
Pro Lys Pro Lys Asn Ala Thr Val Leu Ile Trp
ATT TAT GGT GGT GGT TTT CAA ACT GGA ACA TCA 528
Ile Tyr Gly Gly Gly Phe Gln Thr Gly Thr Ser
TCT TTA CAT GTT TAT GAT GGC AAG TTT CTG GCT 561
Ser Leu His Val Tyr Asp Gly Lys Phe Leu Ala
CGG GTT GAA AGA GTT ATT GTA GTG TCA ATG AAC 594
Arg Val Glu Arg Val Ile Val Val Ser Met Asn
TAT AGG GTG GGT GCC CTA GGA TTC TTA GCT TTG 627
Tyr Arg Val Gly Ala Leu Gly Phe Leu Ala Leu
CCA GGA AAT CCT GAG GCT CCA GGG AAC ATG GGT 660
Pro Gly Asn Pro Glu Ala Pro Gly Asn Met Gly
TTA TTT GAT CAA CAG TTG GCT CTT CAG TGG GTT 693
Leu Phe Asp Gln Gln Leu Ala Leu Gln Trp Val
CAA AAA AAT ATA GCA GCC TTT GGT GGA AAT CCT 726
Gln Lys Asn Ile Ala Ala Phe Gly Gly Asn Pro
AAA AGT GTA ACT CTC TTT GGA GAA AGT GCA GGA 759
Lys Ser Val Thr Leu Phe Gly Glu Ser Ala Gly
GCA GC                                      765
Ala Ala
```

which is part of the sequence of the DNA of claim 1 and operably linked to an expression vector adapted to encode a cholinesterase-like peptide interacting with antibodies to human acetylcholinesterase.

4. The DNA sequence of claim 3 operably linked to an expression vector to form the plasmid FBChEpEx12a (C.N.C.M. Accession No, I-552).

**Claims for the following Contracting State : AT**

1. A process for the production of a DNA sequence of the formula

30 60
CCG TCG ACC CCT GCA TTT CCC CGA AGT ATT TCC CCG AAC TAT TAC ATG ATT TTC ACT CCT
Pro Ser Thr Pro Ala Phe Pro Arg Ser Ile Ser Pro Asn Tyr Tyr Met Ile Phe Thr Pro

90 120
TGC AAA GTT TGC CAT CTT TGT TGC AGA GAA TCG GAA ATC AAT ATG CAT AGC AAA GTC ACA
Cys Lys Val Cys His Leu Cys Cys Arg Glu Ser Glu Ile Asn Met His Ser Lys Val Thr

150 180
ATC ATA TGC ATC AGA TTT CTC TTT TGG TTT GTT TTG CTC TGC ATG CTT ATT GGG AAG TCA
Ile Ile Cys Ile Arg Phe Leu Phe Trp Phe Val Leu Leu Cys Met Leu Ile Gly Lys Ser

210 240
CAT ACT GAA GAT GAC ATC ATA ATT GCA ACA AAG AAT GGA AAA GTC AGA GGG ATG AAC TTG
His Thr Glu Asp Asp Ile Ile Ile Ala Thr Lys Asn Gly Lys Val Arg Gly Met Asn Leu

270 300
ACA GTT TTT GGT GGC ACG GTA ACA GCC TTT CTT GGA ATT CCC TAT GCA CAG CCA CCT CTT
Thr Val Phe Gly Gly Thr Val Thr Ala Phe Leu Gly Ile Pro Tyr Ala Gln Pro Pro Leu

330 360
GGT AGA CTT CGA TTC ACA AAG CCA CAG TCT CTG ACC AGG TGG TCT GAT ATT TGG ACT GCC
Gly Arg Leu Arg Phe Thr Lys Pro Gln Ser Leu Thr Arg Trp Ser Asp Ile Trp Thr Ala

390 420
ACA AAA TAT GCA AAT TCT TGC TGT CAG AAC ATA GAT CAT AGT TTT CCA GGC TTC CAT GGA
Thr Lys Tyr Ala Asn Ser Cys Cys Gln Asn Ile Asp His Ser Phe Pro Gly Phe His Gly

450 480
TCA GAG ATG TGG AAC CCA AAC ACT GAC CTC AGT GAA GAC TGT TTA TAT CTA AAT GTA TGG
Ser Glu Met Trp Asn Pro Asn Thr Asp Leu Ser Glu Asp Cys Leu Tyr Leu Asn Val Trp

510 540
ATT CCA GCA CCT AAA CCA AAA AAT GCC ACT GTA TTG ATA TGG ATT TAT GGT GGT GGT TTT
Ile Pro Ala Pro Lys Pro Lys Asn Ala Thr Val Leu Ile Trp Ile Tyr Gly Gly Gly Phe

570 600
CAA ACT GGA ACA TCA TCT TTA CAT GTT TAT GAT GGC AAG TTT CTG GCT CGG GTT GAA AGA
Gln Thr Gly Thr Ser Ser Leu His Val Tyr Asp Gly Lys Phe Leu Ala Arg Val Glu Arg

630 660
GTT ATT GTA GTG TCA ATG AAC TAT AGG GTG GGT GCC CTA GGA TTC TTA GCT TTG CCA GGA
Val Ile Val Val Ser Met Asn Tyr Arg Val Gly Ala Leu Gly Phe Leu Ala Leu Pro Gly

690 720
AAT CCT GAG GCT CCA GGG AAC ATG GGT TTA TTT GAT CAA CAG TTG GCT CTT CAG TGG GTT
Asn Pro Glu Ala Pro Gly Asn Met Gly Leu Phe Asp Gln Gln Leu Ala Leu Gln Trp Val

750 780
CAA AAA AAT ATA GCA GCC TTT GGT GGA AAT CCT AAA AGT GTA ACT CTC TTT GGA GAA AGT
Gln Lys Asn Ile Ala Ala Phe Gly Gly Asn Pro Lys Ser Val Thr Leu Phe Gly Glu Ser

810 840
GCA GGA GCA GCT TCA GTT AGC CTG CAT TTG CTT TCT CCT GGA AGC CAT TCA TTG TTC ACC
Ala Gly Ala Ala Ser Val Ser Leu His Leu Leu Ser Pro Gly Ser His Ser Leu Phe Thr

870 900
AGA GCC ATT CTG CAA AGT GGA TCC TTT AAT GCT CCT TGG GCG GTA ACA TCT CTT TAT GAA
Arg Ala Ile Leu Gln Ser Gly Ser Phe Asn Ala Pro Trp Ala Val Thr Ser Leu Tyr Glu

```
                                                930                                            960
GCT AGG AAC AGA ACG TTG AAC TTA GCT AAA TTG ACT GGT TGC TCT AGA GAG AAT GAG ACT
Ala Arg Asn Arg Thr Leu Asn Leu Ala Lys Leu Thr Gly Cys Ser Arg Glu Asn Glu Thr

                                                990                                           1020
GAA ATA ATC AAG TGT CTT AGA AAT AAA GAT CCC CAA GAA ATT CTT CTG AAT GAA GCA TTT
Glu Ile Ile Lys Cys Leu Arg Asn Lys Asp Pro Gln Glu Ile Leu Leu Asn Glu Ala Phe

                                               1050                                           1080
GTT GTC CCC TAT GGG ACT CCT TTG TCA GTA AAC TTT GGT CCG ACC GTG GAT GGT GAT TTT
Val Val Pro Tyr Gly Thr Pro Leu Ser Val Asn Phe Gly Pro Thr Val Asp Gly Asp Phe

                                               1110                                           1140
CTC ACT GAC ATG CCA GAC ATA TTA CTT GAA CTT GGA CAA TTT AAA AAA ACC CAG ATT TTG
Leu Thr Asp Met Pro Asp Ile Leu Leu Glu Leu Gly Gln Phe Lys Lys Thr Gln Ile Leu

                                               1170                                           1200
GTG GGT GTT AAT AAA GAT GAA GGG ACA GCT TTT TTA GTC TAT GGT GCT CCT GGC TTC AGC
Val Gly Val Asn Lys Asp Glu Gly Thr Ala Phe Leu Val Tyr Gly Ala Pro Gly Phe Ser

                                               1230                                           1260
AAA GAT AAC ATT AGT ATC ATA ACT AGA AAA GAA TTT CAA GAA GGT TTA AAA ATA TTT TTT
Lys Asp Asn Ile Ser Ile Ile Thr Arg Lys Glu Phe Gln Glu Gly Leu Lys Ile Phe Phe

                                               1290                                           1320
CCA GGA GTG AGT GAG TTT GGA AAG GAA TCC ATC CTT TTT CAA TAC ACA GAC TGG GTA GAT
Pro Gly Val Ser Glu Phe Gly Lys Glu Ser Ile Leu Phe Gln Tyr Thr Asp Trp Val Asp

                                               1350                                           1380
GAT CAA AGA CCT GAA AAC TAC CGT GAG GCC TTG GGT TGT ATG TTG TTG GGG ATT ATA ATT
Asp Gln Arg Pro Glu Asn Tyr Arg Glu Ala Leu Gly Cys Met Leu Leu Gly Ile Ile Ile

                                               1410                                           1440
TCA TAT GCC CTG CCG TTT GAA GTT ACC AAG AAG TTT TCA GAA TGG GGA AAT AAT GCC TTT
Ser Tyr Ala Leu Pro Phe Glu Val Thr Lys Lys Phe Ser Glu Trp Gly Asn Asn Ala Phe

                                               1470                                           1500
TTC TAC TAT TTT GAA CAC CGA TCC TCC AAA CTT CCG TGG CCA GAA TGG ATG GGA GTG ATG
Phe Tyr Tyr Phe Glu His Arg Ser Ser Lys Leu Pro Trp Pro Glu Trp Met Gly Val Met

                                               1530                                           1560
CAT GGC TAT AAA TTG AAT TTG TCT TTG GTT TAC CTC TGG AAA GAA GAG ATA ATT ACA CAA
His Gly Tyr Lys Leu Asn Leu Ser Leu Val Tyr Leu Trp Lys Glu Glu Ile Ile Thr Gln

                                               1590                                           1620
AAT CCT ATT AAA TTT AAG TAC ATC CAT AGT AAA CGG TGG GCA AAT TTT GCA AAA TAT GGG
Asn Pro Ile Lys Phe Lys Tyr Ile His Ser Lys Arg Trp Ala Asn Phe Ala Lys Tyr Gly

                                               1650                                           1680
AAT CCA AAT GAG ACT CAG ACC ATT AGC ACA AGC TGG CCT GTC TTA AAA GCA CTG AAC AAA
Asn Pro Asn Glu Thr Gln Thr Ile Ser Thr Ser Trp Pro Val Leu Lys Ala Leu Asn Lys

                                               1710                                           1740
ATA TCT AAC CTT GAA TAC AGA GTC AAC AAG AAT AAT GAC GAA ACT ACG TGC TCA ACA ATG
Ile Ser Asn Leu Glu Tyr Arg Val Asn Lys Asn Asn Asp Glu Thr Thr Cys Ser Thr Met

                                               1770                                           1800
TCG ATT CTG GAC ATC ATT TTT TCC AAA AGT CTT GGA AAT GAC AGG AAA TAT GAT GAA GCA
Ser Ile Leu Asp Ile Ile Phe Ser Lys Ser Leu Gly Asn Asp Arg Lys Tyr Asp Glu Ala
```

```
                                                   1830                                               1860
GAA TGG GAG TGG AAA GCA GGA TTC CAT CGC TGG AAC AAT TAC ATG ATG GAC TGG AAA AAT
Glu Trp Glu Trp Lys Ala Gly Phe His Arg Trp Asn Asn Tyr Met Met Asp Trp Lys Asn

                                                   1890                                               1920
CAA TTT AAC GAT TAC ACT AGC AAG AAA GAA AGT TGT GTG GGT CTC TAA TTA ATA GAT TTA
Gln Phe Asn Asp Tyr Thr Ser Lys Lys Glu Ser Cys Val Gly Leu End

                                                   1950                                               1980
CCC TTT ATA GAA CAT ATT TTC CTT TAG ATC AAG GCA AAA ATA TCA GGA GCT TTT TTA CAC

                                                   2010                                               2040
ACC TAC TAA AAA AGT TAT TAT GTA GCT GAA ACA CAA ATG CCA GAA GGA TAA TAT TGT TCC

                                                   2070                                               2100
TCA CAT CTT TAA CTT AGT ATT TTA CCA TGC ATT TCA AAA CCC AAA TGG CTA GAA CAT GTT

                                                   2130                                               2160
TAA TTA AAT TTC ACA ATA TAA AGT TCT ACA GTT AAT TAT GTG CAT ATT AAA ACA TGG CCT

                                                   2190                                               2220
GGT TCA ATT TCT TTC TTT CCT TAA TAA ATT TAA GTT TTT TCC CCC CAA AAT TAT CAG TGC

                                                   2250                                               2280
TCT GCT TTT AGT CAC GTG TAT TTT CAT TAC CAC TCG TAA AAA GGT ATC TTT TTT AAA TGA

                                                   2310                                               2340
AGT TAA ATA TTG AAA CAC TGT ACA CCA TAG TTT ACA ATA ATT AGT GTT TCC TAA GTT AAA

                                                   2370                                               2400
ATA AGA ATT GAA TGT CAA TAA TGA GAA TAA TTA AAA TAA GCA CAG AAA ATC ACA AAA AAA

                                                   2430
AAC AAA AAA AAA AAA AAA AAA AAA AAA AAA AAA AAA AAA AAA AAA
```

or part of the sequence encoding a peptide having human pseudocholinesterase activity or operably linked to an expression vector adapted to encode a cholinesterase-like peptide interacting with antibodies to human acetylcholinesterase comprising synthesizing a cDNA containing the DNA sequence or part of the sequence from mRNA.

**2.** The process of claim 1, wherein the DNA sequence encodes a polepeptide having pseudocholinesterase activity.

**3.** The process according to claim 1, wherein the DNA sequence has the formula

```
ATT TCC CCG AAG TAT TAC ATG ATT TTC ACT CCT  33
Ile Ser Pro Lys Tyr Tyr Met Ile Phe Thr Pro
TGC AAA CTT TGC CAT CTT TGT TGC AGA GAA TCG  66
Cys Lys Leu Cys His Leu Cys Cys Arg Glu Ser
GAA ATC AAT ATG CAT AGC AAA GTC ACA ATC ATA  99
Glu Ile Asn Met His Ser Lys Val Thr Ile Ile
TGC ATC AGA TTT CTC TTT TGG TTT CTT TTG CTC 132
Cys Ile Arg Phe Leu Phe Trp Phe Leu Leu Leu
TGC ATG CTT ATT GGG AAG TCA CAT ACT GAA GAT 165
Cys Met Leu Ile Gly Lys Ser His Thr Glu Asp
GAC ATC ATA ATT GCA ACA AAG AAT GGA AAA GTC 198
Asp Ile Ile Ile Ala Thr Lys Asn Gly Lys Val
AGA GGG ATG AAC TTG ACA GTT TTT GGT GGC ACG 231
Arg Gly Met Asn Leu Thr Val Phe Gly Gly Thr
GTA ACA GCC TTT CTT GGA ATT CCC TAT GCA CAG 264
Val Thr Ala Phe Leu Gly Ile Pro Tyr Ala Gln
CCA CCT CTT GGT AGA CTT CGA TTC ACA AAG CCA 297
Pro Pro Leu Gly Arg Leu Arg Phe Thr Lys Pro
CAG TCT CTG ACC AGG TGG TCT GAT ATT TGG ACT 330
Gln Ala Thr Lys Tyr Ala Asn Ser Cys Cys Gln
GCC ACA AAA TAT GCA AAT TCT TGC TGT CAG AAC 363
Ser Leu Thr Arg Trp Ser Asp Ile Trp Thr Asn
ATA GAT CAT AGT TTT CCA GGC TTC CAT GGA TCA 396
Ile Asp His Ser Phe Pro Gly Phe His Gly Ser
GAG ATG TGG AAC CCA AAC ACT GAC CTC AGT GAA 429
Glu Met Trp Asn Pro Asn Thr Asp Leu Ser Glu
GAC TGT TTA TAT CTA AAT GTA TGG ATT CCA GCA 462
Asp Cys Leu Tyr Leu Asn Val Trp Ile Pro Ala
CCT AAA CCA AAA AAT GCC ACT GTA TTG ATA TGG 495
Pro Lys Pro Lys Asn Ala Thr Val Leu Ile Trp
ATT TAT GGT GGT GGT TTT CAA ACT GGA ACA TCA 528
Ile Tyr Gly Gly Gly Phe Gln Thr Gly Thr Ser
TCT TTA CAT GTT TAT GAT GGC AAG TTT CTG GCT 561
Ser Leu His Val Tyr Asp Gly Lys Phe Leu Ala
CGG GTT GAA AGA GTT ATT GTA GTG TCA ATG AAC 594
Arg Val Glu Arg Val Ile Val Val Ser Met Asn
TAT AGG GTG GGT GCC CTA GGA TTC TTA GCT TTG 627
Tyr Arg Val Gly Ala Leu Gly Phe Leu Ala Leu
CCA GGA AAT CCT GAG GCT CCA GGG AAC ATG GGT 660
Pro Gly Asn Pro Glu Ala Pro Gly Asn Met Gly
TTA TTT GAT CAA CAG TTG GCT CTT CAG TGG GTT 693
Leu Phe Asp Gln Gln Leu Ala Leu Gln Trp Val
CAA AAA AAT ATA GCA GCC TTT GGT GGA AAT CCT 726
Gln Lys Asn Ile Ala Ala Phe Gly Gly Asn Pro
AAA AGT GTA ACT CTC TTT GGA GAA AGT GCA GGA 759
Lys Ser Val Thr Leu Phe Gly Glu Ser Ala Gly
GCA GC                                      765
Ala Ala
```

which is part of the sequence of the DNA of claim 1 and operably linked to an expression vector adapted to encode a cholinesterase-like peptide interacting with antibodies to human acetylcholinesterase.

4. The process of claim 3, wherein the DNA sequence is expression vector to form the plasmid FBChEpEx12a (C.N.C.M. Accession No, I-552).

## Patentansprüche

**Patentansprüche für folgende Vertragsstaaten : BE, CH, DE, FR, GB, IT, LI, NL, SE**

1. DNA-Sequenz der Formel

```
                                            30                                              60
CCG TCG ACC CCT GCA TTT CCC CGA AGT ATT TCC CCG AAC TAT TAC ATG ATT TTC ACT CCT
Pro Ser Thr Pro Ala Phe Pro Arg Ser Ile Ser Pro Asn Tyr Tvr Met Ile Phe Thr Pro

                                            90                                             120
TGC AAA GTT TGC CAT CTT TGT TGC AGA GAA TCG GAA ATC AAT ATG CAT AGC AAA GTC ACA
Cys Lys Val Cys His Leu Cys Cys Arg Glu Ser Glu Ile Asn Met His Ser Lys Val Thr

                                           150                                             180
ATC ATA TGC ATC AGA TTT CTC TTT TGG TTT GTT TTG CTC TGC ATG CTT ATT GGG AAG TCA
Ile Ile Cys Ile Arg Phe Leu Phe Trp Phe Val Leu Leu Cys Met Leu Ile Gly Lys Ser

                                           210                                             240
CAT ACT GAA GAT GAC ATC ATA ATT GCA ACA AAG AAT GGA AAA GTC AGA GGG ATG AAC TTG
His Thr Glu Aso Asp Ile Ile Ile Ala Thr Lys Asn Gly Lys Val Arg Gly Met Asn Leu

                                           270                                             300
ACA GTT TTT GGT GGC ACG GTA ACA GCC TTT CTT GGA ATT CCC TAT GCA CAG CCA CCT CTT
Thr Val Phe Gly Gly Thr Val Thr Ala Phe Leu Gly Ile Pro Tyr Ala Gln Pro Pro Leu

                                           330                                             360
GGT AGA CTT CGA TTC ACA AAG CCA CAG TCT CTG ACC AGG TGG TCT GAT ATT TGG ACT GCC
Gly Arg Leu Arg Phe Thr Lys Pro Gln Ser Leu Thr Arg Trp Ser Asp Ile Trp Thr Ala

                                           390                                             420
ACA AAA TAT GCA AAT TCT TGC TGT CAG AAC ATA GAT CAT AGT TTT CCA GGC TTC CAT GGA
Thr Lys Tyr Ala Asn Ser Cys Cys Gln Asn Ile Asp His Ser Phe Pro Gly Phe His Gly

                                           450                                             480
TCA GAG ATG TGG AAC CCA AAC ACT GAC CTC AGT GAA GAC TGT TTA TAT CTA AAT GTA TGG
Ser Glu Met Trp Asn Pro Asn Thr Asp Leu Ser Glu Asp Cys Leu Tyr Leu Asn Val Trp

                                           510                                             540
ATT CCA GCA CCT AAA CCA AAA AAT GCC ACT GTA TTG ATA TGG ATT TAT GGT GGT GGT TTT
Ile Pro Ala Pro Lys Pro Lys Asn Ala Thr Val Leu Ile Trp Ile Tyr Gly Gly Gly Phe

                                           570                                             600
CAA ACT GGA ACA TCA TCT TTA CAT GTT TAT GAT GGC AAG TTT CTG GCT CGG GTT GAA AGA
Gln Thr Glv Thr Ser Ser Leu His Val Tyr Asp Gly Lys Phe Leu Ala Arg Val Glu Arg

                                           630                                             660
GTT ATT GTA GTG TCA ATG AAC TAT AGG GTG GGT GCC CTA GGA TTC TTA GCT TTG CCA GGA
Val Ile Val Val Ser Met Asn Tyr Arg Val Gly Ala Leu Gly Phe Leu Ala Leu Pro Gly

                                           690                                             720
AAT CCT GAG GCT CCA GGG AAC ATG GGT TTA TTT GAT CAA CAG TTG GCT CTT CAG TGG GTT
Asn Pro Glu Ala Pro Gly Asn Met Gly Leu Phe Aso Gln Gln Leu Ala Leu Gln Trp Val

                                           750                                             780
CAA AAA AAT ATA GCA GCC TTT GGT GGA AAT CCT AAA AGT GTA ACT CTC TTT GGA GAA AGT
Gln Lys Asn Ile Ala Ala Phe Gly Gly Asn Pro Lys Ser Val Thr Leu Phe Glv Glu Ser

                                           810                                             840
GCA GGA GCA GCT TCA GTT AGC CTG CAT TTG CTT TCT CCT GGA AGC CAT TCA TTG TTC ACC
Ala Gly Ala Ala Ser Val Ser Leu His Leu Leu Ser Pro Gly Ser His Ser Leu Phe Thr

                                           870                                             900
AGA GCC ATT CTG CAA AGT GGA TCC TTT AAT GCT CCT TGG GCG GTA ACA TCT CTT TAT GAA
Arg Ala Ile Leu Gln Ser Gly Ser Phe Asn Ala Pro Trp Ala Val Thr Ser Leu Tyr Glu
```

```
                                            930                                             960
GCT AGG AAC AGA ACG TTG AAC TTA GCT AAA TTG ACT GGT TGC TCT AGA GAG AAT GAG ACT
Ala Arg Asn Arg Thr Leu Asn Leu Ala Lys Leu Thr Gly Cys Ser Arg Glu Asn Glu Thr

                                            990                                            1020
GAA ATA ATC AAG TGT CTT AGA AAT AAA GAT CCC CAA GAA ATT CTT CTG AAT GAA GCA TTT
Glu Ile Ile Lys Cys Leu Arg Asn Lys Asp Pro Gln Glu Ile Leu Leu Asn Glu Ala Phe

                                           1050                                            1080
GTT GTC CCC TAT GGG ACT CCT TTG TCA GTA AAC TTT GGT CCG ACC GTG GAT GGT GAT TTT
Val Val Pro Tyr Gly Thr Pro Leu Ser Val Asn Phe Gly Pro Thr Val Asp Gly Asp Phe

                                           1110                                            1140
CTC ACT GAC ATG CCA GAC ATA TTA CTT GAA CTT GGA CAA TTT AAA AAA ACC CAG ATT TTG
Leu Thr Asp Met Pro Asp Ile Leu Leu Glu Leu Gly Gln Phe Lys Lys Thr Gln Ile Leu

                                           1170                                            1200
GTG GGT GTT AAT AAA GAT GAA GGG ACA GCT TTT TTA GTC TAT GGT GCT CCT GGC TTC AGC
Val Gly Val Asn Lys Asp Glu Gly Thr Ala Phe Leu Val Tyr Gly Ala Pro Gly Phe Ser

                                           1230                                            1260
AAA GAT AAC ATT AGT ATC ATA ACT AGA AAA GAA TTT CAA GAA GGT TTA AAA ATA TTT TTT
Lys Asp Asn Ile Ser Ile Ile Thr Arg Lys Glu Phe Gln Glu Gly Leu Lys Ile Phe Phe

                                           1290                                            1320
CCA GGA GTG AGT GAG TTT GGA AAG GAA TCC ATC CTT TTT CAA TAC ACA GAC TGG GTA GAT
Pro Gly Val Ser Glu Phe Gly Lys Glu Ser Ile Leu Phe Gln Tyr Thr Asp Trp Val Asp

                                           1350                                            1380
GAT CAA AGA CCT GAA AAC TAC CGT GAG GCC TTG GGT TGT ATG TTG TTG GGG ATT ATA ATT
Asp Gln Arg Pro Glu Asn Tyr Arg Glu Ala Leu Gly Cys Met Leu Leu Gly Ile Ile Ile

                                           1410                                            1440
TCA TAT GCC CTG CCG TTT GAA GTT ACC AAG AAG TTT TCA GAA TGG GGA AAT AAT GCC TTT
Ser Tyr Ala Leu Pro Phe Glu Val Thr Lys Lys Phe Ser Glu Trp Gly Asn Asn Ala Phe

                                           1470                                            1500
TTC TAC TAT TTT GAA CAC CGA TCC TCC AAA CTT CCG TGG CCA GAA TGG ATG GGA GTG ATG
Phe Tyr Tyr Phe Glu His Arg Ser Ser Lys Leu Pro Trp Pro Glu Trp Met Gly Val Met

                                           1530                                            1560
CAT GGC TAT AAA TTG AAT TTG TCT TTG GTT TAC CTC TGG AAA GAA GAG ATA ATT ACA CAA
His Gly Tyr Lys Leu Asn Leu Ser Leu Val Tyr Leu Trp Lys Glu Glu Ile Ile Thr Gln

                                           1590                                            1620
AAT CCT ATT AAA TTT AAG TAC ATC CAT AGT AAA CGG TGG GCA AAT TTT GCA AAA TAT GGG
Asn Pro Ile Lys Phe Lys Tyr Ile His Ser Lys Arg Trp Ala Asn Phe Ala Lys Tyr Gly

                                           1650                                            1680
AAT CCA AAT GAG ACT CAG ACC ATT AGC ACA AGC TGG CCT GTC TTA AAA GCA CTG AAC AAA
Asn Pro Asn Glu Thr Gln Thr Ile Ser Thr Ser Trp Pro Val Leu Lys Ala Leu Asn Lys

                                           1710                                            1740
ATA TCT AAC CTT GAA TAC AGA GTC AAC AAG AAT AAT GAC GAA ACT ACG TGC TCA ACA ATG
Ile Ser Asn Leu Glu Tyr Arg Val Asn Lys Asn Asn Asp Glu Thr Thr Cys Ser Thr Met

                                           1770                                            1800
TCG ATT CTG GAC ATC ATT TTT TCC AAA AGT CTT GGA AAT GAC AGG AAA TAT GAT GAA GCA
Ser Ile Leu Asp Ile Ile Phe Ser Lys Ser Leu Gly Asn Asp Arg Lys Tyr Asp Glu Ala
```

```
                                   1830                                   1860
GAA TGG GAG TGG AAA GCA GGA TTC CAT CGC TGG AAC AAT TAC ATG ATG GAC TGG AAA AAT
Glu Trp Glu Trp Lys Ala Gly Phe His Arg Trp Asn Asn Tyr Met Met Asp Trp Lys Asn

                                   1890                                   1920
CAA TTT AAC GAT TAC ACT AGC AAG AAA GAA AGT TGT GTG GGT CTC TAA TTA ATA GAT TTA
Gln Phe Asn Asp Tyr Thr Ser Lys Lys Glu Ser Cys Val Gly Leu End

                                   1950                                   1980
CCC TTT ATA GAA CAT ATT TTC CTT TAG ATC AAG GCA AAA ATA TCA GGA GCT TTT TTA CAC

                                   2010                                   2040
ACC TAC TAA AAA AGT TAT TAT GTA GCT GAA ACA CAA ATG CCA GAA GGA TAA TAT TGT TCC

                                   2070                                   2100
TCA CAT CTT TAA CTT AGT ATT TTA CCA TGC ATT TCA AAA CCC AAA TGG CTA GAA CAT GTT

                                   2130                                   2160
TAA TTA AAT TTC ACA ATA TAA AGT TCT ACA GTT AAT TAT GTG CAT ATT AAA ACA TGG CCT

                                   2190                                   2220
GGT TCA ATT TCT TTC TTT CCT TAA TAA ATT TAA GTT TTT TCC CCC CAA AAT TAT CAG TGC

                                   2250                                   2280
TCT GCT TTT AGT CAC GTG TAT TTT CAT TAC CAC TCG TAA AAA GGT ATC TTT TTT AAA TGA

                                   2310                                   2340
AGT TAA ATA TTG AAA CAC TGT ACA CCA TAG TTT ACA ATA ATT AGT GTT TCC TAA GTT AAA

                                   2370                                   2400
ATA AGA ATT GAA TGT CAA TAA TGA GAA TAA TTA AAA TAA GCA CAG AAA ATC ACA AAA AAA

                                   2430
AAC AAA AAA AAA AAA AAA AAA AAA AAA AAA AAA AAA AAA AAA AAA
```

oder ein Teil der Sequenz, die ein Peptid mit menschlicher Pseudocholinesteraseaktivität codiert, oder funktionell mit einem Expressionsvektor verbunden ist, der zur Codierung eines Cholinesterase-ähnlichen Peptids, das mit Antikörpern gegen menschliche Acetylcholinesterase eine Wechselwirkung aufweist, angepaßt ist.

**2.** DNA-Sequenz nach Anspruch 1, die ein Polypeptid mit Pseudocholinesteraseaktivität codiert.

**3.** DNA-Sequenz nach Anspruch 1 mit der Formel,

```
ATT TCC CCG AAG TAT TAC ATG ATT TTC ACT CCT  33
Ile Ser Pro Lys Tyr Tyr Met Ile Phe Thr Pro
TGC AAA CTT TGC CAT CTT TGT TGC AGA GAA TCG  66
Cys Lys Leu Cys His Leu Cys Cys Arg Glu Ser
GAA ATC AAT ATG CAT AGC AAA GTC ACA ATC ATA  99
Glu Ile Asn Met His Ser Lys Val Thr Ile Ile
TGC ATC AGA TTT CTC TTT TGG TTT CTT TTG CTC 132
Cys Ile Arg Phe Leu Phe Trp Phe Leu Leu Leu
TGC ATG CTT ATT GGG AAG TCA CAT ACT GAA GAT 165
Cys Met Leu Ile Gly Lys Ser His Thr Glu Asp
GAC ATC ATA ATT GCA ACA AAG AAT GGA AAA GTC 198
Asp Ile Ile Ile Ala Thr Lys Asn Gly Lys Val
AGA GGG ATG AAC TTG ACA GTT TTT GGT GGC ACG 231
Arg Gly Met Asn Leu Thr Val Phe Gly Gly Thr
GTA ACA GCC TTT CTT GGA ATT CCC TAT GCA CAG 264
Val Thr Ala Phe Leu Gly Ile Pro Tyr Ala Gln
CCA CCT CTT GGT AGA CTT CGA TTC ACA AAG CCA 297
Pro Pro Leu Gly Arg Leu Arg Phe Thr Lys Pro
CAG TCT CTG ACC AGG TGG TCT GAT ATT TGG ACT 330
Gln Ala Thr Lys Tyr Ala Asn Ser Cys Cys Gln
GCC ACA AAA TAT GCA AAT TCT TGC TGT CAG AAC 363
Ser Leu Thr Arg Trp Ser Asp Ile Trp Thr Asn
ATA GAT CAT AGT TTT CCA GGC TTC CAT GGA TCA 396
Ile Asp His Ser Phe Pro Gly Phe His Gly Ser
GAG ATG TGG AAC CCA AAC ACT GAC CTC AGT GAA 429
Glu Met Trp Asn Pro Asn Thr Asp Leu Ser Glu
GAC TGT TTA TAT CTA AAT GTA TGG ATT CCA GCA 462
Asp Cys Leu Tyr Leu Asn Val Trp Ile Pro Ala
CCT AAA CCA AAA AAT GCC ACT GTA TTG ATA TGG 495
Pro Lys Pro Lys Asn Ala Thr Val Leu Ile Trp
ATT TAT GGT GGT GGT TTT CAA ACT GGA ACA TCA 528
Ile Tyr Gly Gly Gly Phe Gln Thr Gly Thr Ser
TCT TTA CAT GTT TAT GAT GGC AAG TTT CTG GCT 561
Ser Leu His Val Tyr Asp Gly Lys Phe Leu Ala
CGG GTT GAA AGA GTT ATT GTA GTG TCA ATG AAC 594
Arg Val Glu Arg Val Ile Val Val Ser Met Asn
TAT AGG GTG GGT GCC CTA GGA TTC TTA GCT TTG 627
Tyr Arg Val Gly Ala Leu Gly Phe Leu Ala Leu
CCA GGA AAT CCT GAG GCT CCA GGG AAC ATG GGT 660
Pro Gly Asn Pro Glu Ala Pro Gly Asn Met Gly
TTA TTT GAT CAA CAG TTG GCT CTT CAG TGG GTT 693
Leu Phe Asp Gln Gln Leu Ala Leu Gln Trp Val
CAA AAA AAT ATA GCA GCC TTT GGT GGA AAT CCT 726
Gln Lys Asn Ile Ala Ala Phe Gly Gly Asn Pro
AAA AGT GTA ACT CTC TTT GGA GAA AGT GCA GGA 759
Lys Ser Val Thr Leu Phe Gly Glu Ser Ala Gly
GCA GC                                      765
Ala Ala
```

die ein Teil der Sequenz der DNA von Anspruch 1 ist und funktionell mit einem Expressionsvektor verbunden ist, der zur Codierung eines Cholinesterase-ähnlichen Peptids, das mit Antikörpern gegen menschliche Acetylcholinesterase eine Wechselwirkung aufweist, angepaßt ist.

**4.** DNA-Sequenz nach Anspruch 3, die funktionell mit einem Expressionsvektor zur Erzeugung des Plasmids FBChEpEx12a (C.N.C.M. Hinterlegungsnummer I-552) verbunden ist.

## Patentansprüche für folgende Vertragsstaat : AT

**1.** Verfahren zur Herstellung einer DNA-Sequenz der Formel

```
                                             30                                                60
CCG TCG ACC CCT GCA TTT CCC CGA AGT ATT TCC CCG AAC TAT TAC ATG ATT TTC ACT CCT
Pro Ser Thr Pro Ala Phe Pro Arg Ser Ile Ser Pro Asn Tyr Tyr Met Ile Phe Thr Pro

                                             90                                               120
TGC AAA GTT TGC CAT CTT TGT TGC AGA GAA TCG GAA ATC AAT ATG CAT AGC AAA GTC ACA
Lys Lys Val Cys His Leu Cys Cys Arg Glu Ser Glu Ile Asn Met His Ser Lys Val Thr

                                            150                                               180
ATC ATA TGC ATC AGA TTT CTC TTT TGG TTT GTT TTG CTC TGC ATG CTT ATT GGG AAG TCA
Ile Ile Cys Ile Arg Phe Leu Phe Trp Phe Val Leu Leu Cys Met Leu Ile Gly Lys Ser

                                            210                                               240
CAT ACT GAA GAT GAC ATC ATA ATT GCA ACA AAG AAT GGA AAA GTC AGA GGG ATG AAC TTG
His Thr Glu Asp Asp Ile Ile Ile Ala Thr Lys Asn Gly Lys Val Arg Gly Met Asn Leu

                                            270                                               300
ACA GTT TTT GGT GGC ACG GTA ACA GCC TTT CTT GGA ATT CCC TAT GCA CAG CCA CCT CTT
Thr Val Phe Gly Gly Thr Val Thr Ala Phe Leu Gly Ile Pro Tyr Ala Gln Pro Pro Leu

                                            330                                               360
GGT AGA CTT CGA TTC ACA AAG CCA CAG TCT CTG ACC AGG TGG TCT GAT ATT TGG ACT GCC
Gly Arg Leu Arg Phe Thr Lys Pro Gln Ser Leu Thr Arg Trp Ser Asp Ile Trp Thr Ala

                                            390                                               420
ACA AAA TAT GCA AAT TCT TGC TGT CAG AAC ATA GAT CAT AGT TTT CCA GGC TTC CAT GGA
Thr Lys Tyr Ala Asn Ser Cys Cys Gln Asn Ile Asp His Ser Phe Pro Gly Phe His Gly

                                            450                                               480
TCA GAG ATG TGG AAC CCA AAC ACT GAC CTC AGT GAA GAC TGT TTA TAT CTA AAT GTA TGG
Ser Glu Met Trp Asn Pro Asn Thr Asp Leu Ser Glu Asp Cys Leu Tyr Leu Asn Val Trp

                                            510                                               540
ATT CCA GCA CCT AAA CCA AAA AAT GCC ACT GTA TTG ATA TGG ATT TAT GGT GGT GGT TTT
Ile Pro Ala Pro Lys Pro Lys Asn Ala Thr Val Leu Ile Trp Ile Tyr Gly Gly Gly Phe

                                            570                                               600
CAA ACT GGA ACA TCA TCT TTA CAT GTT TAT GAT GGC AAG TTT CTG GCT CGG GTT GAA AGA
Gln Thr Gly Thr Ser Ser Leu His Val Tyr Asp Gly Lys Phe Leu Ala Arg Val Glu Arg

                                            630                                               660
GTT ATT GTA GTG TCA ATG AAC TAT AGG GTG GGT GCC CTA GGA TTC TTA GCT TTG CCA GGA
Val Ile Val Val Ser Met Asn Tyr Arg Val Gly Ala Leu Gly Phe Leu Ala Leu Pro Gly

                                            690                                               720
AAT CCT GAG GCT CCA GGG AAC ATG GGT TTA TTT GAT CAA CAG TTG GCT CTT CAG TGG GTT
Asn Pro Glu Ala Pro Gly Asn Met Gly Leu Phe Asp Gln Gln Leu Ala Leu Gln Trp Val

                                            750                                               780
CAA AAA AAT ATA GCA GCC TTT GGT GGA AAT CCT AAA AGT GTA ACT CTC TTT GGA GAA AGT
Gln Lys Asn Ile Ala Ala Phe Gly Gly Asn Pro Lys Ser Val Thr Leu Phe Gly Glu Ser

                                            810                                               840
GCA GGA GCA GCT TCA GTT AGC CTG CAT TTG CTT TCT CCT GGA AGC CAT TCA TTG TTC ACC
Ala Gly Ala Ala Ser Val Ser Leu His Leu Leu Ser Pro Gly Ser His Ser Leu Phe Thr

                                            870                                               900
AGA GCC ATT CTG CAA AGT GGA TCC TTT AAT GCT CCT TGG GCG GTA ACA TCT CTT TAT GAA
Arg Ala Ile Leu Gln Ser Gly Ser Phe Asn Ala Pro Trp Ala Val Thr Ser Leu Tyr Glu
```

```
                                                  930                                                  960
GCT AGG AAC AGA ACG TTG AAC TTA GCT AAA TTG ACT GGT TGC TCT AGA GAG AAT GAG ACT
Ala Arg Asn Arg Thr Leu Asn Leu Ala Lys Leu Thr Gly Cys Ser Arg Glu Asn Glu Thr

                                                  990                                                 1020
GAA ATA ATC AAG TGT CTT AGA AAT AAA GAT CCC CAA GAA ATT CTT CTG AAT GAA GCA TTT
Glu Ile Ile Lys Cys Leu Arg Asn Lys Asp Pro Gln Glu Ile Leu Leu Asn Glu Ala Phe

                                                 1050                                                 1080
GTT GTC CCC TAT GGG ACT CCT TTG TCA GTA AAC TTT GGT CCG ACC GTG GAT GGT GAT TTT
Val Val Pro Tyr Gly Thr Pro Leu Ser Val Asn Phe Gly Pro Thr Val Asp Gly Asp Phe

                                                 1110                                                 1140
CTC ACT GAC ATG CCA GAC ATA TTA CTT GAA CTT GGA CAA TTT AAA AAA ACC CAG ATT TTG
Leu Thr Asp Met Pro Asp Ile Leu Leu Glu Leu Gly Gln Phe Lys Lys Thr Gln Ile Leu

                                                 1170                                                 1200
GTG GGT GTT AAT AAA GAT GAA GGG ACA GCT TTT TTA GTC TAT GGT GCT CCT GGC TTC AGC
Val Gly Val Asn Lys Asp Glu Gly Thr Ala Phe Leu Val Tyr Gly Ala Pro Gly Phe Ser

                                                 1230                                                 1260
AAA GAT AAC ATT AGT ATC ATA ACT AGA AAA GAA TTT CAA GAA GGT TTA AAA ATA TTT TTT
Lys Asp Asn Ile Ser Ile Ile Thr Arg Lys Glu Phe Gln Glu Gly Leu Lys Ile Phe Phe

                                                 1290                                                 1320
CCA GGA GTG AGT GAG TTT GGA AAG GAA TCC ATC CTT TTT CAA TAC ACA GAC TGG GTA GAT
Pro Gly Val Ser Glu Phe Gly Lys Glu Ser Ile Leu Phe Gln Tyr Thr Asp Trp Val Asp

                                                 1350                                                 1380
GAT CAA AGA CCT GAA AAC TAC CGT GAG GCC TTG GGT TGT ATG TTG TTG GGG ATT ATA ATT
Asp Gln Arg Pro Glu Asn Tyr Arg Glu Ala Leu Gly Cys Met Leu Leu Gly Ile Ile Ile

                                                 1410                                                 1440
TCA TAT GCC CTG CCG TTT GAA GTT ACC AAG AAG TTT TCA GAA TGG GGA AAT AAT GCC TTT
Ser Tyr Ala Leu Pro Phe Glu Val Thr Lys Lys Phe Ser Glu Trp Gly Asn Asn Ala Phe

                                                 1470                                                 1500
TTC TAC TAT TTT GAA CAC CGA TCC TCC AAA CTT CCG TGG CCA GAA TGG ATG GGA GTG ATG
Phe Tyr Tyr Phe Glu His Arg Ser Ser Lys Leu Pro Trp Pro Glu Trp Met Gly Val Met

                                                 1530                                                 1560
CAT GGC TAT AAA TTG AAT TTG TCT TTG GTT TAC CTC TGG AAA GAA GAG ATA ATT ACA CAA
His Gly Tyr Lys Leu Asn Leu Ser Leu Val Tyr Leu Trp Lys Glu Glu Ile Ile Thr Gln

                                                 1590                                                 1620
AAT CCT ATT AAA TTT AAG TAC ATC CAT AGT AAA CGG TGG GCA AAT TTT GCA AAA TAT GGG
Asn Pro Ile Lys Phe Lys Tyr Ile His Ser Lys Arg Trp Ala Asn Phe Ala Lys Tyr Gly

                                                 1650                                                 1680
AAT CCA AAT GAG ACT CAG ACC ATT AGC ACA AGC TGG CCT GTC TTA AAA GCA CTG AAC AAA
Asn Pro Asn Glu Thr Gln Thr Ile Ser Thr Ser Trp Pro Val Leu Lys Ala Leu Asn Lys

                                                 1710                                                 1740
ATA TCT AAC CTT GAA TAC AGA GTC AAC AAG AAT AAT GAC GAA ACT ACG TGC TCA ACA ATG
Ile Ser Asn Leu Glu Tyr Arg Val Asn Lys Asn Asn Asp Glu Thr Thr Cys Ser Thr Met

                                                 1770                                                 1800
TCG ATT CTG GAC ATC ATT TTT TCC AAA AGT CTT GGA AAT GAC AGG AAA TAT GAT GAA GCA
Ser Ile Leu Asp Ile Ile Phe Ser Lys Ser Leu Gly Asn Asp Arg Lys Tyr Asp Glu Ala
```

1830 1860
GAA TGG GAG TGG AAA GCA GGA TTC CAT CGC TGG AAC AAT TAC ATG ATG GAC TGG AAA AAT
Glu Trp Glu Trp Lys Ala Gly Phe His Arg Trp Asn Asn Tyr Met Met Asp Trp Lys Asn

1890 1920
CAA TTT AAC GAT TAC ACT AGC AAG AAA GAA AGT TGT GTG GGT CTC TAA TTA ATA GAT TTA
Gln Phe Asn Asp Tyr Thr Ser Lys Lys Glu Ser Cys Val Gly Leu End

1950 1980
CCC TTT ATA GAA CAT ATT TTC CTT TAG ATC AAG GCA AAA ATA TCA GGA GCT TTT TTA CAC

2010 2040
ACC TAC TAA AAA AGT TAT TAT GTA GCT GAA ACA CAA ATG CCA GAA GGA TAA TAT TGT TCC

2070 2100
TCA CAT CTT TAA CTT AGT ATT TTA CCA TGC ATT TCA AAA CCC AAA TGG CTA GAA CAT GTT

2130 2160
TAA TTA AAT TTC ACA ATA TAA AGT TCT ACA GTT AAT TAT GTG CAT ATT AAA ACA TGG CCT

2190 2220
GGT TCA ATT TCT TTC TTT CCT TAA TAA ATT TAA GTT TTT TCC CCC CAA AAT TAT CAG TGC

2250 2280
TCT GCT TTT AGT CAC GTG TAT TTT CAT TAC CAC TCG TAA AAA GGT ATC TTT TTT AAA TGA

2310 2340
AGT TAA ATA TTG AAA CAC TGT ACA CCA TAG TTT ACA ATA ATT AGT GTT TCC TAA GTT AAA

2370 2400
ATA AGA ATT GAA TGT CAA TAA TGA GAA TAA TTA AAA TAA GCA CAG AAA ATC ACA AAA AAA

2430
AAC AAA AAA AAA AAA AAA AAA AAA AAA AAA AAA AAA AAA AAA AAA

oder eines Teiles der Sequenz, die ein Peptid mit menschlicher Pseudocholinesteraseaktivität codiert, oder funktionell mit einem Expressionsvektor verbunden ist, der zur Codierung eines Cholinesterase-ähnlichen Peptids, das mit Antikörpern gegen menschliche Acetylcholinesterase eine Wechselwirkung aufweist, angepaßt ist, wobei das Verfahren die Synthese einer die DNA-Sequenz oder einen Teil der Sequenz enthaltenden cDNA aus mRNA umfaßt.

**2.** Verfahren nach Anspruch 1, wobei die DNA-Sequenz ein Polypeptid mit Pseudocholinesteraseaktivität codiert.

**3.** Verfahren nach Anspruch 1, wobei die DNA-Sequenz die Formel,

```
ATT TCC CCG AAG TAT TAC ATG ATT TTC ACT CCT  33
Ile Ser Pro Lys Tyr Tyr Met Ile Phe Thr Pro
TGC AAA CTT TGC CAT CTT TGT TGC AGA GAA TCG  66
Cys Lys Leu Cys His Leu Cys Cys Arg Glu Ser
GAA ATC AAT ATG CAT AGC AAA GTC ACA ATC ATA  99
Glu Ile Asn Met His Ser Lys Val Thr Ile Ile
TGC ATC AGA TTT CTC TTT TGG TTT CTT TTG CTC 132
Cys Ile Arg Phe Leu Phe Trp Phe Leu Leu Leu
TGC ATG CTT ATT GGG AAG TCA CAT ACT GAA GAT 165
Cys Met Leu Ile Gly Lys Ser His Thr Glu Asp
GAC ATC ATA ATT GCA ACA AAG AAT GGA AAA GTC 198
Asp Ile Ile Ile Ala Thr Lys Asn Gly Lys Val
AGA GGG ATG AAC TTG ACA GTT TTT GGT GGC ACG 231
Arg Gly Met Asn Leu Thr Val Phe Gly Gly Thr
GTA ACA GCC TTT CTT GGA ATT CCC TAT GCA CAG 264
Val Thr Ala Phe Leu Gly Ile Pro Tyr Ala Gln
CCA CCT CTT GGT AGA CTT CGA TTC ACA AAG CCA 297
Pro Pro Leu Gly Arg Leu Arg Phe Thr Lys Pro
CAG TCT CTG ACC AGG TGG TCT GAT ATT TGG ACT 330
Gln Ala Thr Lys Tyr Ala Asn Ser Cys Cys Gln
GCC ACA AAA TAT GCA AAT TCT TGC TGT CAG AAC 363
Ser Leu Thr Arg Trp Ser Asp Ile Trp Thr Asn
ATA GAT CAT AGT TTT CCA GGC TTC CAT GGA TCA 396
Ile Asp His Ser Phe Pro Gly Phe His Gly Ser
GAG ATG TGG AAC CCA AAC ACT GAC CTC AGT GAA 429
Glu Met Trp Asn Pro Asn Thr Asp Leu Ser Glu
GAC TGT TTA TAT CTA AAT GTA TGG ATT CCA GCA 462
Asp Cys Leu Tyr Leu Asn Val Trp Ile Pro Ala
CCT AAA CCA AAA AAT GCC ACT GTA TTG ATA TGG 495
Pro Lys Pro Lys Asn Ala Thr Val Leu Ile Trp
ATT TAT GGT GGT GGT TTT CAA ACT GGA ACA TCA 528
Ile Tyr Gly Gly Gly Phe Gln Thr Gly Thr Ser
TCT TTA CAT GTT TAT GAT GGC AAG TTT CTG GCT 561
Ser Leu His Val Tyr Asp Gly Lys Phe Leu Ala
CGG GTT GAA AGA GTT ATT GTA GTG TCA ATG AAC 594
Arg Val Glu Arg Val Ile Val Val Ser Met Asn
TAT AGG GTG GGT GCC CTA GGA TTC TTA GCT TTG 627
Tyr Arg Val Gly Ala Leu Gly Phe Leu Ala Leu
CCA GGA AAT CCT GAG GCT CCA GGG AAC ATG GGT 660
Pro Gly Asn Pro Glu Ala Pro Gly Asn Met Gly
TTA TTT GAT CAA CAG TTG GCT CTT CAG TGG GTT 693
Leu Phe Asp Gln Gln Leu Ala Leu Gln Trp Val
CAA AAA AAT ATA GCA GCC TTT GGT GGA AAT CCT 726
Gln Lys Asn Ile Ala Ala Phe Gly Gly Asn Pro
AAA AGT GTA ACT CTC TTT GGA GAA AGT GCA GGA 759
Lys Ser Val Thr Leu Phe Gly Glu Ser Ala Gly
GCA GC                                      765
Ala Ala
```

aufweist, die ein Teil der Sequenz der DNA von Anspruch 1 ist und funktionell mit einem Expressionsvektor verbunden ist, der zur Codierung eines Cholinesterase-ähnlichen Peptids, das mit Antikörpern gegen menschliche Acetylcholinesterase eine Wechselwirkung aufweist, angepaßt ist.

4. Verfahren nach Anspruch 3, wobei die DNA-Sequenz funktionell mit einem Expressionsvektor zur Erzeugung des Plasmids FBChEpEx12a (C.N.C.M. Hinterlegungsnummer I-552) verbunden ist.

## Revendications

**Revendications pour les Etats contractants suivants : BE, CH, DE, FR, GB, IT, LI, NL, SE**

1. Séquence d'ADN de formule

```
                                            30                                                  60
CCG TCG ACC CCT GCA TTT CCC CGA AGT ATT TCC CCG AAC TAT TAC ATG ATT TTC ACT CCT
Pro Ser Thr Pro Ala Phe Pro Arg Ser Ile Ser Pro Asn Tyr Tyr Met Ile Phe Thr Pro

                                            90                                                 120
TGC AAA GTT TGC CAT CTT TGT TGC AGA GAA TCG GAA ATC AAT ATG CAT AGC AAA GTC ACA
Cys Lys Val Cys His Leu Cys Cys Arg Glu Ser Glu Ile Asn Met His Ser Lys Val Thr

                                           150                                                 180
ATC ATA TGC ATC AGA TTT CTC TTT TGG TTT GTT TTG CTC TGC ATG CTT ATT GGG AAG TCA
Ile Ile Cys Ile Arg Phe Leu Phe Trp Phe Val Leu Leu Cys Met Leu Ile Gly Lys Ser

                                           210                                                 240
CAT ACT GAA GAT GAC ATC ATA ATT GCA ACA AAG AAT GGA AAA GTC AGA GGG ATG AAC TTG
His Thr Glu Aso Asp Ile Ile Ile Ala Thr Lys Asn Gly Lys Val Arg Gly Met Asn Leu

                                           270                                                 300
ACA GTT TTT GGT GGC ACG GTA ACA GCC TTT CTT GGA ATT CCC TAT GCA CAG CCA CCT CTT
Thr Val Phe Gly Gly Thr Val Thr Ala Phe Leu Gly Ile Pro Tyr Ala Gln Pro Pro Leu

                                           330                                                 360
GGT AGA CTT CGA TTC ACA AAG CCA CAG TCT CTG ACC AGG TGG TCT GAT ATT TGG ACT GCC
Gly Arg Leu Arg Phe Thr Lys Pro Gln Ser Leu Thr Arg Trp Ser Asp Ile Trp Thr Ala

                                           390                                                 420
ACA AAA TAT GCA AAT TCT TGC TGT CAG AAC ATA GAT CAT AGT TTT CCA GGC TTC CAT GGA
Thr Lys Tyr Ala Asn Ser Cys Cys Gln Asn Ile Asp His Ser Phe Pro Gly Phe His Gly

                                           450                                                 480
TCA GAG ATG TGG AAC CCA AAC ACT GAC CTC AGT GAA GAC TGT TTA TAT CTA AAT GTA TGG
Ser Glu Met Trp Asn Pro Asn Thr Asp Leu Ser Glu Asp Cys Leu Tyr Leu Asn Val Trp

                                           510                                                 540
ATT CCA GCA CCT AAA CCA AAA AAT GCC ACT GTA TTG ATA TGG ATT TAT GGT GGT GGT TTT
Ile Pro Ala Pro Lys Pro Lys Asn Ala Thr Val Leu Ile Trp Ile Tyr Gly Gly Gly Phe

                                           570                                                 600
CAA ACT GGA ACA TCA TCT TTA CAT GTT TAT GAT GGC AAG TTT CTG GCT CGG GTT GAA AGA
Gln Thr Gly Thr Ser Ser Leu His Val Tyr Asp Gly Lys Phe Leu Ala Arg Val Glu Arg

                                           630                                                 660
GTT ATT GTA GTG TCA ATG AAC TAT AGG GTG GGT GCC CTA GGA TTC TTA GCT TTG CCA GGA
Val Ile Val Val Ser Met Asn Tyr Arg Val Gly Ala Leu Gly Phe Leu Ala Leu Pro Gly

                                           690                                                 720
AAT CCT GAG GCT CCA GGG AAC ATG GGT TTA TTT GAT CAA CAG TTG GCT CTT CAG TGG GTT
Asn Pro Glu Ala Pro Gly Asn Met Gly Leu Phe Aso Gln Gln Leu Ala Leu Gln Trp Val

                                           750                                                 780
CAA AAA AAT ATA GCA GCC TTT GGT GGA AAT CCT AAA AGT GTA ACT CTC TTT GGA GAA AGT
Gln Lys Asn Ile Ala Ala Phe Gly Gly Asn Pro Lys Ser Val Thr Leu Phe Gly Glu Ser

                                           810                                                 840
GCA GGA GCA GCT TCA GTT AGC CTG CAT TTG CTT TCT CCT GGA AGC CAT TCA TTG TTC ACC
Ala Gly Ala Ala Ser Val Ser Leu His Leu Leu Ser Pro Gly Ser His Ser Leu Phe Thr

                                           870                                                 900
AGA GCC ATT CTG CAA AGT GGA TCC TTT AAT GCT CCT TGG GCG GTA ACA TCT CTT TAT GAA
Arg Ala Ile Leu Gln Ser Gly Ser Phe Asn Ala Pro Trp Ala Val Thr Ser Leu Tyr Glu
```

```
                                                      930                                                   960
GCT AGG AAC AGA ACG TTG AAC TTA GCT AAA TTG ACT GGT TGC TCT AGA GAG AAT GAG ACT
Ala Arg Asn Arg Thr Leu Asn Leu Ala Lys Leu Thr Gly Cys Ser Arg Glu Asn Glu Thr

                                                      990                                                  1020
GAA ATA ATC AAG TGT CTT AGA AAT AAA GAT CCC CAA GAA ATT CTT CTG AAT GAA GCA TTT
Glu Ile Ile Lys Cys Leu Arg Asn Lys Asp Pro Gln Glu Ile Leu Leu Asn Glu Ala Phe

                                                     1050                                                  1080
GTT GTC CCC TAT GGG ACT CCT TTG TCA GTA AAC TTT GGT CCG ACC GTG GAT GGT GAT TTT
Val Val Pro Tyr Gly Thr Pro Leu Ser Val Asn Phe Gly Pro Thr Val Asp Gly Asp Phe

                                                     1110                                                  1140
CTC ACT GAC ATG CCA GAC ATA TTA CTT GAA CTT GGA CAA TTT AAA AAA ACC CAG ATT TTG
Leu Thr Asp Met Pro Asp Ile Leu Leu Glu Leu Gly Gln Phe Lys Lys Thr Gln Ile Leu

                                                     1170                                                  1200
GTG GGT GTT AAT AAA GAT GAA GGG ACA GCT TTT TTA GTC TAT GGT GCT CCT GGC TTC AGC
Val Gly Val Asn Lys Asp Glu Gly Thr Ala Phe Leu Val Tyr Gly Ala Pro Gly Phe Ser

                                                     1230                                                  1260
AAA GAT AAC ATT AGT ATC ATA ACT AGA AAA GAA TTT CAA GAA GGT TTA AAA ATA TTT TTT
Lys Asp Asn Ile Ser Ile Ile Thr Arg Lys Glu Phe Gln Glu Gly Leu Lys Ile Phe Phe

                                                     1290                                                  1320
CCA GGA GTG AGT GAG TTT GGA AAG GAA TCC ATC CTT TTT CAA TAC ACA GAC TGG GTA GAT
Pro Gly Val Ser Glu Phe Gly Lys Glu Ser Ile Leu Phe Gln Tyr Thr Asp Trp Val Asp

                                                     1350                                                  1380
GAT CAA AGA CCT GAA AAC TAC CGT GAG GCC TTG GGT TGT ATG TTG TTG GGG ATT ATA ATT
Asp Gln Arg Pro Glu Asn Tyr Arg Glu Ala Leu Gly Cys Met Leu Leu Gly Ile Ile Ile

                                                     1410                                                  1440
TCA TAT GCC CTG CCG TTT GAA GTT ACC AAG AAG TTT TCA GAA TGG GGA AAT AAT GCC TTT
Ser Tyr Ala Leu Pro Phe Glu Val Thr Lys Lys Phe Ser Glu Trp Gly Asn Asn Ala Phe

                                                     1470                                                  1500
TTC TAC TAT TTT GAA CAC CGA TCC TCC AAA CTT CCG TGG CCA GAA TGG ATG GGA GTG ATG
Phe Tyr Tyr Phe Glu His Arg Ser Ser Lys Leu Pro Trp Pro Glu Trp Met Gly Val Met

                                                     1530                                                  1560
CAT GGC TAT AAA TTG AAT TTG TCT TTG GTT TAC CTC TGG AAA GAA GAG ATA ATT ACA CAA
His Gly Tyr Lys Leu Asn Leu Ser Leu Val Tyr Leu Trp Lys Glu Glu Ile Ile Thr Gln

                                                     1590                                                  1620
AAT CCT ATT AAA TTT AAG TAC ATC CAT AGT AAA CGG TGG GCA AAT TTT GCA AAA TAT GGG
Asn Pro Ile Lys Phe Lys Tyr Ile His Ser Lys Arg Trp Ala Asn Phe Ala Lys Tyr Gly

                                                     1650                                                  1680
AAT CCA AAT GAG ACT CAG ACC ATT AGC ACA AGC TGG CCT GTC TTA AAA GCA CTG AAC AAA
Asn Pro Asn Glu Thr Gln Thr Ile Ser Thr Ser Trp Pro Val Leu Lys Ala Leu Asn Lys

                                                     1710                                                  1740
ATA TCT AAC CTT GAA TAC AGA GTC AAC AAG AAT AAT GAC GAA ACT ACG TGC TCA ACA ATG
Ile Ser Asn Leu Glu Tyr Arg Val Asn Lys Asn Asn Asp Glu Thr Thr Cys Ser Thr Met

                                                     1770                                                  1800
TCG ATT CTG GAC ATC ATT TTT TCC AAA AGT CTT GGA AAT GAC AGG AAA TAT GAT GAA GCA
Ser Ile Leu Asp Ile Ile Phe Ser Lys Ser Leu Gly Asn Asp Arg Lys Tyr Asp Glu Ala
```

```
                                           1830                                          1860
GAA TGG GAG TGG AAA GCA GGA TTC CAT CGC TGG AAC AAT TAC ATG ATG GAC TGG AAA AAT
Glu Trp Glu Trp Lys Ala Gly Phe His Arg Trp Asn Asn Tyr Met Met Asp Trp Lys Asn

                                           1890                                          1920
CAA TTT AAC GAT TAC ACT AGC AAG AAA GAA AGT TGT GTG GGT CTC TAA TTA ATA GAT TTA
Gln Phe Asn Asp Tyr Thr Ser Lys Lys Glu Ser Cys Val Gly Leu End

                                           1950                                          1980
CCC TTT ATA GAA CAT ATT TTC CTT TAG ATC AAG GCA AAA ATA TCA GGA GCT TTT TTA CAC

                                           2010                                          2040
ACC TAC TAA AAA AGT TAT TAT GTA GCT GAA ACA CAA ATG CCA GAA GGA TAA TAT TGT TCC

                                           2070                                          2100
TCA CAT CTT TAA CTT AGT ATT TTA CCA TGC ATT TCA AAA CCC AAA TGG CTA GAA CAT GTT

                                           2130                                          2160
TAA TTA AAT TTC ACA ATA TAA AGT TCT ACA GTT AAT TAT GTG CAT ATT AAA ACA TGG CCT

                                           2190                                          2220
GGT TCA ATT TCT TTC TTT CCT TAA TAA ATT TAA GTT TTT TCC CCC CAA AAT TAT CAG TGC

                                           2250                                          2280
TCT GCT TTT AGT CAC GTG TAT TTT CAT TAC CAC TCG TAA AAA GGT ATC TTT TTT AAA TGA

                                           2310                                          2340
AGT TAA ATA TTG AAA CAC TGT ACA CCA TAG TTT ACA ATA ATT AGT GTT TCC TAA GTT AAA

                                           2370                                          2400
ATA AGA ATT GAA TGT CAA TAA TGA GAA TAA TTA AAA TAA GCA CAG AAA ATC ACA AAA AAA

                                           2430
AAC AAA AAA AAA AAA AAA AAA AAA AAA AAA AAA AAA AAA AAA AAA
```

ou une partie de la séquence codant pour un peptide ayant une activité de pseudocholinestérase humaine ou liée opérationnellement à un vecteur d'expression adapté pour coder pour un peptide analogue à la cholinestérase réagissant mutuellement avec des anticorps d'acétylcholinestérase humaine.

**2.** Séquence d'ADN suivant la revendication 1, codant pour un peptide ayant une activité de pseudocholinestérase.

**3.** Séquence d'ADN suivant la revendication 1, de formule

```
ATT TCC CCG AAG TAT TAC ATG ATT TTC ACT CCT   33
Ile Ser Pro Lys Tyr Tyr Met Ile Phe Thr Pro
TGC AAA CTT TGC CAT CTT TGT TGC AGA GAA TCG   66
Cys Lys Leu Cys His Leu Cys Cys Arg Glu Ser
GAA ATC AAT ATG CAT AGC AAA GTC ACA ATC ATA   99
Glu Ile Asn Met His Ser Lys Val Thr Ile Ile
TGC ATC AGA TTT CTC TTT TGG TTT CTT TTG CTC  132
Cys Ile Arg Phe Leu Phe Trp Phe Leu Leu Leu
TGC ATG CTT ATT GGG AAG TCA CAT ACT GAA GAT  165
Cys Met Leu Ile Gly Lys Ser His Thr Glu Asp
GAC ATC ATA ATT GCA ACA AAG AAT GGA AAA GTC  198
Asp Ile Ile Ile Ala Thr Lys Asn Gly Lys Val
AGA GGG ATG AAC TTG ACA GTT TTT GGT GGC ACG  231
Arg Gly Met Asn Leu Thr Val Phe Gly Gly Thr
GTA ACA GCC TTT CTT GGA ATT CCC TAT GCA CAG  264
Val Thr Ala Phe Leu Gly Ile Pro Tyr Ala Gln
CCA CCT CTT GGT AGA CTT CGA TTC ACA AAG CCA  297
Pro Pro Leu Gly Arg Leu Arg Phe Thr Lys Pro
CAG TCT CTG ACC AGG TGG TCT GAT ATT TGG ACT  330
Gln Ala Thr Lys Tyr Ala Asn Ser Cys Cys Gln
GCC ACA AAA TAT GCA AAT TCT TGC TGT CAG AAC  363
Ser Leu Thr Arg Trp Ser Asp Ile Trp Thr Asn
ATA GAT CAT AGT TTT CCA GGC TTC CAT GGA TCA  396
Ile Asp His Ser Phe Pro Gly Phe His Gly Ser
GAG ATG TGG AAC CCA AAC ACT GAC CTC AGT GAA  429
Glu Met Trp Asn Pro Asn Thr Asp Leu Ser Glu
GAC TGT TTA TAT CTA AAT GTA TGG ATT CCA GCA  462
Asp Cys Leu Tyr Leu Asn Val Trp Ile Pro Ala
CCT AAA CCA AAA AAT GCC ACT GTA TTG ATA TGG  495
Pro Lys Pro Lys Asn Ala Thr Val Leu Ile Trp
ATT TAT GGT GGT GGT TTT CAA ACT GGA ACA TCA  528
Ile Tyr Gly Gly Gly Phe Gln Thr Gly Thr Ser
TCT TTA CAT GTT TAT GAT GGC AAG TTT CTG GCT  561
Ser Leu His Val Tyr Asp Gly Lys Phe Leu Ala
CGG GTT GAA AGA GTT ATT GTA GTG TCA ATG AAC  594
Arg Val Glu Arg Val Ile Val Val Ser Met Asn
TAT AGG GTG GGT GCC CTA GGA TTC TTA GCT TTG  627
Tyr Arg Val Gly Ala Leu Gly Phe Leu Ala Leu
CCA GGA AAT CCT GAG GCT CCA GGG AAC ATG GGT  660
Pro Gly Asn Pro Glu Ala Pro Gly Asn Met Gly
TTA TTT GAT CAA CAG TTG GCT CTT CAG TGG GTT  693
Leu Phe Asp Gln Gln Leu Ala Leu Gln Trp Val
CAA AAA AAT ATA GCA GCC TTT GGT GGA AAT CCT  726
Gln Lys Asn Ile Ala Ala Phe Gly Gly Asn Pro
AAA AGT GTA ACT CTC TTT GGA GAA AGT GCA GGA  759
Lys Ser Val Thr Leu Phe Gly Glu Ser Ala Gly
GCA GC                                       765
Ala Ala
```

qui est une partie de la séquence de l'ADN de la revendication 1 et liée opérationnellement à un vecteur d'expression adapté pour coder pour un peptide analogue à la cholinestérase réagissant mutuellement avec des anticorps d'acétylcholinestérase humaine.

4. Séquence d'ADN suivant la revendication 3, liée opérationnellement à un vecteur d'expression pour former le plasmide FBChEpEx12a (C.N.C.M. numéro d'accès I-552).

**Revendications pour l'Etat contractant suivant : AT**

1. Procédé pour la production d'une séquence d'ADN de formule

```
                                            30                                                60
CCG TCG ACC CCT GCA TTT CCC CGA AGT ATT TCC CCG AAC TAT TAC ATG ATT TTC ACT CCT
Pro Ser Thr Pro Ala Phe Pro Arg Ser Ile Ser Pro Asn Tyr Tvr Met Ile Phe Thr Pro

                                            90                                               120
TGC AAA GTT TGC CAT CTT TGT TGC AGA GAA TCG GAA ATC AAT ATG CAT AGC AAA GTC ACA
Cys Lys Val Cys His Leu Cys Cys Arg Glu Ser Glu Ile Asn Met His Ser Lys Val Thr

                                           150                                               180
ATC ATA TGC ATC AGA TTT CTC TTT TGG TTT GTT TTG CTC TGC ATG CTT ATT GGG AAG TCA
Ile Ile Cys Ile Arg Phe Leu Phe Trp Phe Val Leu Leu Cys Met Leu Ile Gly Lys Ser

                                           210                                               240
CAT ACT GAA GAT GAC ATC ATA ATT GCA ACA AAG AAT GGA AAA GTC AGA GGG ATG AAC TTG
His Thr Glu Aso Asp Ile Ile Ile Ala Thr Lys Asn Gly Lys Val Arg Gly Met Asn Leu

                                           270                                               300
ACA GTT TTT GGT GGC ACG GTA ACA GCC TTT CTT GGA ATT CCC TAT GCA CAG CCA CCT CTT
Thr Val Phe Gly Gly Thr Val Thr Ala Phe Leu Gly Ile Pro Tyr Ala Gln Pro Pro Leu

                                           330                                               360
GGT AGA CTT CGA TTC ACA AAG CCA CAG TCT CTG ACC AGG TGG TCT GAT ATT TGG ACT GCC
Gly Arg Leu Arg Phe Thr Lys Pro Gln Ser Leu Thr Arg Trp Ser Asp Ile Trp Thr Ala

                                           390                                               420
ACA AAA TAT GCA AAT TCT TGC TGT CAG AAC ATA GAT CAT AGT TTT CCA GGC TTC CAT GGA
Thr Lys Tyr Ala Asn Ser Cys Cys Gln Asn Ile Asp His Ser Phe Pro Gly Phe His Gly

                                           450                                               480
TCA GAG ATG TGG AAC CCA AAC ACT GAC CTC AGT GAA GAC TGT TTA TAT CTA AAT GTA TGG
Ser Glu Met Trp Asn Pro Asn Thr Asp Leu Ser Glu Asp Cys Leu Tyr Leu Asn Val Trp

                                           510                                               540
ATT CCA GCA CCT AAA CCA AAA AAT GCC ACT GTA TTG ATA TGG ATT TAT GGT GGT GGT TTT
Ile Pro Ala Pro Lys Pro Lys Asn Ala Thr Val Leu Ile Trp Ile Tyr Gly Gly Gly Phe

                                           570                                               600
CAA ACT GGA ACA TCA TCT TTA CAT GTT TAT GAT GGC AAG TTT CTG GCT CGG GTT GAA AGA
Gln Thr Gly Thr Ser Ser Leu His Val Tyr Asp Gly Lys Phe Leu Ala Arg Val Glu Arg

                                           630                                               660
GTT ATT GTA GTG TCA ATG AAC. TAT AGG GTG GGT GCC CTA GGA TTC TTA GCT TTG CCA GGA
Val Ile Val Val Ser Met Asn Tyr Arg Val Gly Ala Leu Gly Phe Leu Ala Leu Pro Gly

                                           690                                               720
AAT CCT GAG GCT CCA GGG AAC ATG GGT TTA TTT GAT CAA CAG TTG GCT CTT CAG TGG GTT
Asn Pro Glu Ala Pro Gly Asn Met Gly Leu Phe Asp Gln Gln Leu Ala Leu Gln Trp Val

                                           750                                               780
CAA AAA AAT ATA GCA GCC TTT GGT GGA AAT CCT AAA AGT GTA ACT CTC TTT GGA GAA AGT
Gln Lys Asn Ile Ala.Ala Phe Gly Gly Asn Pro Lys Ser Val Thr Leu Phe Gly Glu Ser

                                           810                                               840
GCA GGA GCA GCT TCA GTT AGC CTG CAT TTG CTT TCT CCT GGA AGC CAT TCA TTG TTC ACC
Ala Gly Ala Ala Ser Val Ser Leu His Leu Leu Ser Pro Gly Ser His Ser Leu Phe Thr

                                           870                                               900
AGA GCC ATT CTG CAA AGT GGA TCC TTT AAT GCT CCT TGG GCG GTA ACA TCT CTT TAT GAA
Arg Ala Ile Leu Gln Ser Gly Ser Phe Asn Ala Pro Trp Ala Val Thr Ser Leu Tyr Glu
```

930 960
GCT AGG AAC AGA ACG TTG AAC TTA GCT AAA TTG ACT GGT TGC TCT AGA GAG AAT GAG ACT
Ala Arg Asn Arg Thr Leu Asn Leu Ala Lys Leu Thr Gly Cys Ser Arg Glu Asn Glu Thr

990 1020
GAA ATA ATC AAG TGT CTT AGA AAT AAA GAT CCC CAA GAA ATT CTT CTG AAT GAA GCA TTT
Glu Ile Ile Lys Cys Leu Arg Asn Lys Asp Pro Gln Glu Ile Leu Leu Asn Glu Ala Phe

1050 1080
GTT GTC CCC TAT GGG ACT CCT TTG TCA GTA AAC TTT GGT CCG ACC GTG GAT GGT GAT TTT
Val Val Pro Tyr Gly Thr Pro Leu Ser Val Asn Phe Gly Pro Thr Val Asp Gly Asp Phe

1110 1140
CTC ACT GAC ATG CCA GAC ATA TTA CTT GAA CTT GGA CAA TTT AAA AAA ACC CAG ATT TTG
Leu Thr Asp Met Pro Asp Ile Leu Leu Glu Leu Gly Gln Phe Lys Lys Thr Gln Ile Leu

1170 1200
GTG GGT GTT AAT AAA GAT GAA GGG ACA GCT TTT TTA GTC TAT GGT GCT CCT GGC TTC AGC
Val Gly Val Asn Lys Asp Glu Gly Thr Ala Phe Leu Val Tyr Gly Ala Pro Gly Phe Ser

1230 1260
AAA GAT AAC ATT AGT ATC ATA ACT AGA AAA GAA TTT CAA GAA GGT TTA AAA ATA TTT TTT
Lys Asp Asn Ile Ser Ile Ile Thr Arg Lys Glu Phe Gln Glu Gly Leu Lys Ile Phe Phe

1290 1320
CCA GGA GTG AGT GAG TTT GGA AAG GAA TCC ATC CTT TTT CAA TAC ACA GAC TGG GTA GAT
Pro Gly Val Ser Glu Phe Gly Lys Glu Ser Ile Leu Phe Gln Tyr Thr Asp Trp Val Asp

1350 1380
GAT CAA AGA CCT GAA AAC TAC CGT GAG GCC TTG GGT TGT ATG TTG TTG GGG ATT ATA ATT
Asp Gln Arg Pro Glu Asn Tyr Arg Glu Ala Leu Gly Cys Met Leu Leu Gly Ile Ile Ile

1410 1440
TCA TAT GCC CTG CCG TTT GAA GTT ACC AAG AAG TTT TCA GAA TGG GGA AAT AAT GCC TTT
Ser Tyr Ala Leu Pro Phe Glu Val Thr Lys Lys Phe Ser Glu Trp Gly Asn Asn Ala Phe

1470 1500
TTC TAC TAT TTT GAA CAC CGA TCC TCC AAA CTT CCG TGG CCA GAA TGG ATG GGA GTG ATG
Phe Tyr Tyr Phe Glu His Arg Ser Ser Lys Leu Pro Trp Pro Glu Trp Met Gly Val Met

1530 1560
CAT GGC TAT AAA TTG AAT TTG TCT TTG GTT TAC CTC TGG AAA GAA GAG ATA ATT ACA CAA
His Gly Tyr Lys Leu Asn Leu Ser Leu Val Tyr Leu Trp Lys Glu Glu Ile Ile Thr Gln

1590 1620
AAT CCT ATT AAA TTT AAG TAC ATC CAT AGT AAA CGG TGG GCA AAT TTT GCA AAA TAT GGG
Asn Pro Ile Lys Phe Lys Tyr Ile His Ser Lys Arg Trp Ala Asn Phe Ala Lys Tyr Gly

1650 1680
AAT CCA AAT GAG ACT CAG ACC ATT AGC ACA AGC TGG CCT GTC TTA AAA GCA CTG AAC AAA
Asn Pro Asn Glu Thr Gln Thr Ile Ser Thr Ser Trp Pro Val Leu Lys Ala Leu Asn Lys

1710 1740
ATA TCT AAC CTT GAA TAC AGA GTC AAC AAG AAT AAT GAC GAA ACT ACG TGC TCA ACA ATG
Ile Ser Asn Leu Glu Tyr Arg Val Asn Lys Asn Asn Asp Glu Thr Thr Cys Ser Thr Met

1770 1800
TCG ATT CTG GAC ATC ATT TTT TCC AAA AGT CTT GGA AAT GAC AGG AAA TAT GAT GAA GCA
Ser Ile Leu Asp Ile Ile Phe Ser Lys Ser Leu Gly Asn Asp Arg Lys Tyr Asp Glu Ala

```
                                                 1830                                          1860
GAA TGG GAG TGG AAA GCA GGA TTC CAT CGC TGG AAC AAT TAC ATG ATG GAC TGG AAA AAT
Glu Trp Glu Trp Lys Ala Gly Phe His Arg Trp Asn Asn Tyr Met Met Asp Trp Lys Asn

                                                 1890                                          1920
CAA TTT AAC GAT TAC ACT AGC AAG AAA GAA AGT TGT GTG GGT CTC TAA TTA ATA GAT TTA
Gln Phe Asn Asp Tyr Thr Ser Lys Lys Glu Ser Cys Val Gly Leu End

                                                 1950                                          1980
CCC TTT ATA GAA CAT ATT TTC CTT TAG ATC AAG GCA AAA ATA TCA GGA GCT TTT TTA CAC

                                                 2010                                          2040
ACC TAC TAA AAA AGT TAT TAT GTA GCT GAA ACA CAA ATG CCA GAA GGA TAA TAT TGT TCC

                                                 2070                                          2100
TCA CAT CTT TAA CTT AGT ATT TTA CCA TGC ATT TCA AAA CCC AAA TGG CTA GAA CAT GTT

                                                 2130                                          2160
TAA TTA AAT TTC ACA ATA TAA AGT TCT ACA GTT AAT TAT GTG CAT ATT AAA ACA TGG CCT

                                                 2190                                          2220
GGT TCA ATT TCT TTC TTT CCT TAA TAA ATT TAA GTT TTT TCC CCC CAA AAT TAT CAG TEC

                                                 2250                                          2280
TCT GCT TTT AGT CAC GTG TAT TTT CAT TAC CAC TCG TAA AAA GGT ATC TTT TTT AAA TGA

                                                 2310                                          2340
AGT TAA ATA TTG AAA CAC TGT ACA CCA TAG TTT ACA ATA ATT AGT GTT TCC TAA GTT AAA

                                                 2370                                          2400
ATA AGA ATT GAA TGT CAA TAA TGA GAA TAA TTA AAA TAA GCA CAG AAA A1C ACA AAA AAA

                                                 2430
AAC AAA AAA AAA AAA AAA AAA AAA AAA AAA AAA AAA AAA AAA AAA
```

ou une partie de la séquence codant pour un peptide ayant une activité de pseudocholinestérase humaine ou liée opérationnellement à un vecteur d'expression adapté pour coder pour un peptide analogue à la cholinestérase réagissant mutuellement avec des anticorps d'acétylcholinestérase humaine, ledit procédé comprenant la synthèse d'un cADN contenant la séquence d'ADN ou une partie de la séquence à partir de mARN.

**2.** Procédé suivant la revendication 1, dans lequel la séquence d'ADN code pour un peptide ayant une activité de pseudocholinestérase.

**3.** Procédé suivant la revendication 1, dans lequel la séquence d'ADN a pour formule

```
ATT TCC CCG AAG TAT TAC ATG ATT TTC ACT CCT  33
Ile Ser Pro Lys Tyr Tyr Met Ile Phe Thr Pro
TGC AAA CTT TGC CAT CTT TGT TGC AGA GAA TCG  66
Cys Lys Leu Cys His Leu Cys Cys Arg Glu Ser
GAA ATC AAT ATG CAT AGC AAA GTC ACA ATC ATA  99
Glu Ile Asn Met His Ser Lys Val Thr Ile Ile
TGC ATC AGA TTT CTC TTT TGG TTT CTT TTG CTC 132
Cys Ile Arg Phe Leu Phe Trp Phe Leu Leu Leu
TGC ATG CTT ATT GGG AAG TCA CAT ACT GAA GAT 165
Cys Met Leu Ile Gly Lys Ser His Thr Glu Asp
GAC ATC ATA ATT GCA ACA AAG AAT GGA AAA GTC 198
Asp Ile Ile Ile Ala Thr Lys Asn Gly Lys Val
AGA GGG ATG AAC TTG ACA GTT TTT GGT GGC ACG 231
Arg Gly Met Asn Leu Thr Val Phe Gly Gly Thr
GTA ACA GCC TTT CTT GGA ATT CCC TAT GCA CAG 264
Val Thr Ala Phe Leu Gly Ile Pro Tyr Ala Gln
CCA CCT CTT GGT AGA CTT CGA TTC ACA AAG CCA 297
Pro Pro Leu Gly Arg Leu Arg Phe Thr Lys Pro
CAG TCT CTG ACC AGG TGG TCT GAT ATT TGG ACT 330
Gln Ala Thr Lys Tyr Ala Asn Ser Cys Cys Gln
GCC ACA AAA TAT GCA AAT TCT TGC TGT CAG AAC 363
Ser Leu Thr Arg Trp Ser Asp Ile Trp Thr Asn
ATA GAT CAT AGT TTT CCA GGC TTC CAT GGA TCA 396
Ile Asp His Ser Phe Pro Gly Phe His Gly Ser
GAG ATG TGG AAC CCA AAC ACT GAC CTC AGT GAA 429
Glu Met Trp Asn Pro Asn Thr Asp Leu Ser Glu
GAC TGT TTA TAT CTA AAT GTA TGG ATT CCA GCA 462
Asp Cys Leu Tyr Leu Asn Val Trp Ile Pro Ala
CCT AAA CCA AAA AAT GCC ACT GTA TTG ATA TGG 495
Pro Lys Pro Lys Asn Ala Thr Val Leu Ile Trp
ATT TAT GGT GGT GGT TTT CAA ACT GGA ACA TCA 528
Ile Tyr Gly Gly Gly Phe Gln Thr Gly Thr Ser
TCT TTA CAT GTT TAT GAT GGC AAG TTT CTG GCT 561
Ser Leu His Val Tyr Asp Gly Lys Phe Leu Ala
CGG GTT GAA AGA GTT ATT GTA GTG TCA ATG AAC 594
Arg Val Glu Arg Val Ile Val Val Ser Met Asn
TAT AGG GTG GGT GCC CTA GGA TTC TTA GCT TTG 627
Tyr Arg Val Gly Ala Leu Gly Phe Leu Ala Leu
CCA GGA AAT CCT GAG GCT CCA GGG AAC ATG GGT 660
Pro Gly Asn Pro Glu Ala Pro Gly Asn Met Gly
TTA TTT GAT CAA CAG TTG GCT CTT CAG TGG GTT 693
Leu Phe Asp Gln Gln Leu Ala Leu Gln Trp Val
CAA AAA AAT ATA GCA GCC TTT GGT GGA AAT CCT 726
Gln Lys Asn Ile Ala Ala Phe Gly Gly Asn Pro
AAA AGT GTA ACT CTC TTT GGA GAA AGT GCA GGA 759
Lys Ser Val Thr Leu Phe Gly Glu Ser Ala Gly
GCA GC                                      765
Ala Ala
```

qui est une partie de la séquence de l'ADN de la revendication 1 et liée opérationnellement à un vecteur d'expression adapté pour coder pour un peptide analogue à la cholinestérase réagissant mutuellement avec des anticorps d'acétylcholinestérase humaine.

**4.** Procédé suivant la revendication 3, dans lequel la séquence d'ADN est liée opérationnellement à un vecteur d'expression pour former le plasmide FBChEpEx12a (C.N.C.M. numéro d'accès I-552).